(19) Europäisches Patentamt
European Patent Office
Office européen des brevets

(11) Veröffentlichungsnummer : **0 634 399 A1**

# (12) EUROPÄISCHE PATENTANMELDUNG

(21) Anmeldenummer : **94810397.3**

(22) Anmeldetag : **05.07.94**

(51) Int. Cl.[6] : **C07D 211/46,** C07D 405/12, C08K 5/3435

(30) Priorität : **13.07.93 CH 2097/93**

(43) Veröffentlichungstag der Anmeldung : **18.01.95 Patentblatt 95/03**

(84) Benannte Vertragsstaaten : **BE DE ES FR GB IT NL**

(71) Anmelder : **CIBA-GEIGY AG**
**Klybeckstrasse 141**
**CH-4002 Basel (CH)**

(72) Erfinder : **Steinmann, Alfred, Dr.**
**Les Russilles**
**CH-1724 Praroman (CH)**

## (54) Addukte von gehinderten Amin-Epoxiden als Stabilisatoren.

(57) Es werden Ester oder Phenolether der Formel I oder II beschrieben.

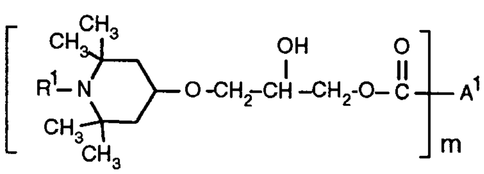

(I)

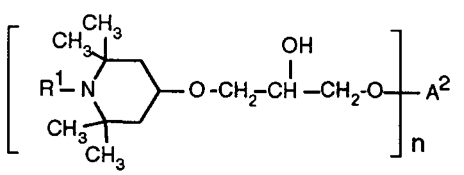

,

(II)

worin m und n jeweils eine ganze Zahl aus dem Bereich von 1 bis 6 darstellen ;
$A^1$ einen m-wertigen Kohlenwasserstoffrest mit 1 bis 30 Kohlenstoffatomen darstellt ; oder einen m-wertigen Kohlenwasserstoffrest mit 2 bis 30 Kohlenstoffatomen darstellt, der die Heteroatome Sauerstoff, Stickstoff und/oder Schwefel enthält und dessen freie Valenzen an Kohlenstoffatomen lokalisiert sind ; wobei $A^1$ im Fall m = 2 zusätzlich eine direkte Bindung umfaßt ;
$A^2$ einen n-wertigen aromatischen oder araliphatischen Kohlenwasserstoffrest mit 6 bis 30 Kohlenstoffatomen darstellt ; oder einen n-wertigen aromatischen oder araliphatischen Kohlenwasserstoffrest mit 5 bis 30 Kohlenstoffatomen darstellt, der die Heteroatome Sauerstoff, Stickstoff und/oder Schwefel enthält und dessen n freie Valenzen an solchen Kohlenstoffatomen lokalisiert sind, die Bestandteil aromatischer Ringe sind ;
$R^1$ Wasserstoff ; einen Kohlenwasserstoff- oder Kohlenwasserstoffoxyrest mit 1 bis 36 Kohlenstoffatomen, der unsubstituiert oder durch $-CO-N(R^3)_2$ substituiert oder durch $-CO-N(R^3)-$ oder $-N(R^3)-CO-$ oder 1 bis 6 Sauerstoff- oder Schwefelatome unterbrochen ist ; Benzoyl oder Naphtoyl, welches jeweils durch 1 bis 3 $C_1$-$C_4$-Alkyl- oder $C_1$-$C_4$-Alkoxyreste substituiert ist ; oder $-CO-R^2$ bedeutet ; wobei
$R^2$ $C_1$-$C_{18}$-Alkyl ; $C_5$-$C_8$-Cycloalkyl ; Phenyl ; Naphthyl ; $C_7$-$C_9$-Phenylalkyl ; oder $C_{11}$-$C_{14}$-Naphthylalkyl bedeutet ; und $R^3$ dieselben Bedeutungen umfaßt wie $R^2$ oder Wasserstoff bedeutet.

Die Verbindungen eignen sich zur Stabilisierung organischen Materials gegen den schädigenden Einfluß von Licht, Sauerstoff und/oder Wärme.

EP 0 634 399 A1

Die Erfindung betrifft neue Verbindungen, die durch Umsetzung Epoxigruppen-haltiger gehinderter Amine mit Phenolen oder Carbonsäuren erhalten werden können, deren Verwendung als Stabilisatoren organischen Materials gegen den schädigenden Einfluß von Licht, Sauerstoff und/oder Wärme, sowie die entsprechenden stabilisierten Zusammensetzungen.

Die Herstellung einiger Verbindungen vom Typ des 2,2,6,6-Tetramethyl-4-(2,3-epoxypropoxy)-piperidin sowie ihre Verwendung als Stabilisatoren für organische Polymere wird beispielsweise durch Luston und Vass, Makromol. Chem., Macromol. Symp. 27, 231 (1989), beschrieben.

Reaktionsprodukte dieser Epoxide mit Toluolsulfonsäure und Ethylendisulfonsäure, sowie deren kombinierte Verwendung als Härtungskatalysatoren und Lichtschutzmittel in Lacken sind in der EP-A-097616 genannt.

Die Bindung reaktiver Polyalkylpiperidine an Fluorpolymere, die freie Carboxygruppen enthalten, wird in EP-A-526 399 beschrieben.

Die Publikation EP-A-001835 lehrt die weitere Umsetzung der Epoxygruppen-haltigen Piperidine mit Dicarbonsäureanhydriden zu Polyestern.

Es besteht weiterhin Bedarf an neuen Stabilisatoren vom Typ des Tetraalkyl-4-(2,3-epoxypropoxy)-piperidins mit verbesserten Gebrauchseigenschaften.

Gegenstand der Erfindung sind daher zunächst Ester der Formel I

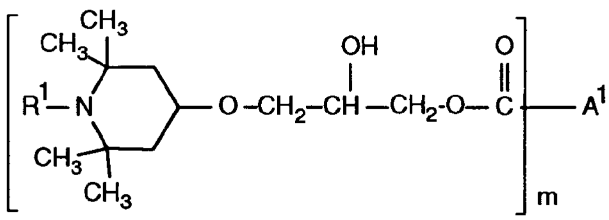

(I)

oder Phenolether der Formel II

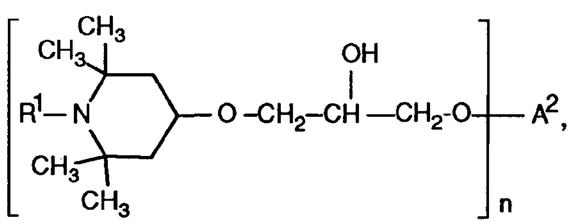

(II)

worin m und n jeweils eine ganze Zahl aus dem Bereich von 1 bis 6 darstellen;
$A^1$ einen m-wertigen Kohlenwasserstoffrest mit 1 bis 30 Kohlenstoffatomen darstellt; oder einen m-wertigen Kohlenwasserstoffrest mit 2 bis 30 Kohlenstoffatomen darstellt, der die Heteroatome Sauerstoff, Stickstoff und/oder Schwefel enthält und dessen freie Valenzen an Kohlenstoffatomen lokalisiert sind; wobei $A^1$ im Fall m = 2 zusätzlich eine direkte Bindung umfaßt;
$A^2$ einen n-wertigen aromatischen oder araliphatischen Kohlenwasserstoffrest mit 6 bis 30 Kohlenstoffatomen darstellt; oder einen n-wertigen aromatischen oder araliphatischen Kohlenwasserstoffrest mit 5 bis 30 Kohlenstoffatomen darstellt, der die Heteroatome Sauerstoff, Stickstoff und/oder Schwefel enthält und dessen n freie Valenzen an solchen Kohlenstoffatomen lokalisiert sind, die Bestandteil aromatischer Ringe sind;
$R^1$ Wasserstoff; einen Kohlenwasserstoff- oder Kohlenwasserstoffoxyrest mit 1 bis 36 Kohlenstoffatomen, der unsubstituiert oder durch $-CO-N(R^3)_2$ substituiert oder durch $-CO-N(R^3)-$ oder $-N(R^3)-CO-$ oder 1 bis 6 Sauerstoff- oder Schwefelatome unterbrochen ist; Benzoyl oder Naphtoyl, welches jeweils durch 1 bis 3 $C_1$-$C_4$-Alkyl- oder $C_1$-$C_4$-Alkoxyreste substituiert ist; oder $-CO-R^2$ bedeutet; wobei $R^2$ $C_1$-$C_{18}$-Alkyl; $C_5$-$C_8$-Cycloalkyl; Phenyl; Naphthyl; $C_7$-$C_9$-Phenylalkyl; oder $C_{11}$-$C_{14}$-Naphthylalkyl bedeutet; und $R^3$ dieselben Bedeutungen umfaßt wie $R^2$ oder Wasserstoff bedeutet.

Stellt $A^1$ einen m-wertigen Kohlenwasserstoffrest mit 2 bis 30 Kohlenstoffatomen dar, der die Heteroatome Sauerstoff, Stickstoff und/oder Schwefel enthält, so liegen diese in der Regel in Form von -O-, tertiär -OH, $-N(R^2)-$,

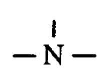

und/oder -S- vor; stellt $A^2$ einen n-wertigen aromatischen oder araliphatischen Kohlenwasserstoffrest mit 5 bis 30 Kohlenstoffatomen dar, der die Heteroatome Sauerstoff, Stickstoff und/oder Schwefel enthält, so liegen diese in der Regel in Form von -O-, tertiär -OH, -N($R^2$)-,

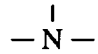

und/oder -S- vor. Aryl bedeutet einen aromatischen Kohlenwasserstoffrest wie beispielsweise Phenyl oder Naphthyl. Aralkyl bedeutet Alkyl, das durch einen aromatischen Kohlenwasserstoffrest, z.B. einen $C_6$-$C_{10}$-Kohlenwasserstoffrest, substituiert ist; Beispiele dafür sind neben anderen Benzyl oder α-Methylbenzyl.

Die genannten zwei- und dreibindigen Heteroatome können an dasselbe Nachbaratom oder an verschiedene Nachbaratome binden; die Nachbaratome sind in der Regel Kohlenstoffatome. So kann zum Beispiel -O- einen Ether oder eine Carbonylgruppe bilden. Entsprechendes gilt für -S- und

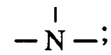;

so kann

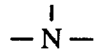

zum Beispiel Bestandteil eines tertiären aliphatischen Amins, einer Ringstruktur, beispielsweise Pyridin, oder einer Cyanogruppe sein.

Unter einer tertiären Hydroxylgruppe ist ein Substituent des Typs -OH an einem tertiären Kohlenstoffatom zu verstehen. Dieses zeichnet sich dadurch aus, daß es mit seinen übrigen drei freien Valenzen an drei weitere Kohlenstoffatome bindet; die resultierenden Verbindungen werden auch als tertiäre Alkohole bezeichnet (vergl. Beyer/Walter, Lehrbuch der Organischen Chemie, 20. Auflage, S.59 und 111, S. Hirzel Verlag, Stuttgart 1984). Ein Rest, welcher eine tertiäre Hydroxylgruppe enthält, enthält daher mindestens 4 Kohlenstoffatome.

Enthalten $A^1$, $A^2$ oder $R^1$ im Rahmen ihrer angegebenen Bedeutungen Alkyl oder Alkylen, welches durch -O-, -S-,

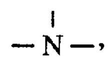,

-CO-N($R^3$)-, -N($R^3$)CO- oder -N($R^2$)- unterbrochen ist, so handelt es sich um Alkyl beziehungsweise Alkylen mit mindestens 2, vorzugsweise mindestens 4 Kohlenstoffatomen, das bevorzugt durch 1-6 Gruppen -O- oder -S- oder 1-3 Gruppen

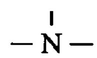

und/oder -N($R^2$)-, besonders durch 1-6 -O-, oder 1-2 -S-,

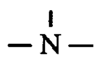

oder -N($R^2$)- unterbrochen ist; die Heteroatome oder Carbonylgruppen binden vorzugsweise an gesättigte Kohlenstoffatome und nicht an andere Heteroatome oder Carbonylgruppen; Peroxo- oder Hydrazinstrukturen treten in der Regel nicht auf. Eine Bedeutungsmöglichkeit dieser Reste sind beispielsweise Polyoxyethylenketten, deren Enden mit $C_1$-$C_8$-Alkyl abgesättigt sind.

Die Verbindungen der Formel I und II lassen sich vorteilhaft zur Stabilisierung organischen Materials gegen den schädigenden Einfluß von Licht, Sauerstoff und/oder Wärme einsetzen.

Zwei gleichartige Reste in derselben Formel können dabei gleiche oder verschiedene Bedeutungen haben; beispielsweise kann einer der beiden Reste $R^3$ in der Teilformel -CO-N($R^3$)$_2$ Wasserstoff bedeuten, während der andere Rest $R^3$ eine der Bedeutungen von $R^2$ hat.

$A^1$ als monovalenter Rest umfaßt beispielsweise die folgenden Bedeutungen:
Verzweigtes oder unverzweigtes Alkyl wie Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl, Pentakosyl oder Triakosyl; bevorzugt ist unverzweigtes Alkyl, besonders bevorzugt unverzweigtes $C_6$-$C_{18}$-Alkyl;
verzweigtes oder unverzweigtes $C_2$-$C_{30}$-Alkenyl wie Vinyl, α-Methylvinyl, Propenyl (Allyl), Butenyl, Pentenyl,

Hexenyl, Heptenyl, Octenyl, Nonenyl, Decenyl, Dodecenyl, Pentadecenyl, Octadecenyl;
durch $C_5$-$C_8$-Cycloalkyl substituiertes Alkyl wie Cyclopentylmethyl, Cyclohexylmethyl, Cycloheptylmethyl, Cyclooctylmethyl, Cyclohexylethyl, 2-Cyclohexyl-n-propyl, 3-Cyclohexyl-n-propyl, 4-Cyclohexyl-n-butyl;
durch $C_5$-$C_8$-Cycloalkyl oder eine oder mehrere, bevorzugt 1-3, der Gruppen -S-, -O- und/oder -$NR^2$- unterbrochenes $C_2$-$C_{25}$-Alkyl, beispielsweise der Formeln

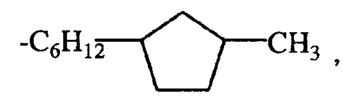 ,

-$CH_2$-S-$C_4H_9$, -$C_2H_4$-O-$C_2H_4$-O-$C_{12}H_{25}$, -$C_{18}H_{36}$-N($C_4H_9$)$_2$; $C_5$-$C_8$-Cycloalkyl oder $C_6$-$C_8$-Cycloalkenyl, das unsubstituiert oder mit $C_1$-$C_{12}$-Alkyl oder $C_2$-$C_{12}$-Alkenyl substituiert ist, wie Cyclopentyl, Cyclohexyl, Cycloheptyl, Cyclooctyl, 2- oder 4-Methylcyclohexyl, Dimethylcyclohexyl, Trimethylcyclohexyl, t-Butylcyclohexyl, 2-Cyclohexenyl, 3-Cycloheptenyl, Cyclooctatetraenyl, 4-tert.-Butyl-cyclohex-2-enyl, bevorzugt Cyclohexyl und Cyclohexenyl, besonders Cyclohexyl;
$C_6$-$C_{10}$-Bicycloalkenyl wie z.B. Bicyclo-(2,2,1)-hepta-5-en-2-yl;
$C_7$-$C_{12}$-Phenylalkyl, $C_7$-$C_{12}$-Phenylalkenyl, $C_{11}$-$C_{16}$-Naphthylalkyl, $C_{11}$-$C_{16}$-Naphthylalkenyl, $C_{13}$-$C_{18}$-Biphenylalkyl oder $C_{13}$-$C_{18}$-Biphenylalkenyl wie beispielsweise $C_1$-$C_6$-Alkyl oder $C_2$-$C_6$-Alkenyl, das mit Phenyl, Naphthyl oder Biphenyl substituiert ist wie z.B. Benzyl, Phenethyl, β-Phenylvinyl, 3-Phenylpropyl, α-Methylbenzyl, α,α-Dimethylbenzyl, 2-Phenylethenyl, 1-Phenylprop-2-enyl, 6-Phenylhexyl, 1-Naphthylmethyl, 2-Naphthylmethyl, 1-Naphthyleth-1-yl, 2-(4-Biphenylyl)-prop-2-yl, 1-(4-Biphenylyl)-pent-3-en-1-yl.

$A^1$ und $A^2$ als monovalente araliphatische oder aromatische Reste umfassen beispielsweise die folgenden Bedeutungen:
Phenyl, Naphthyl, Biphenyl, Triazinyl, 2,4-Diphenyl-1,3,5-triazin-6-yl-phenyl, Benzoylphenyl, Benzylphenyl, α-Methylbenzylphenyl, α,α-Dimethylbenzylphenyl;
mit $C_1$-$C_{12}$-Alkyl oder $C_2$-$C_{12}$-Alkenyl substituiertes Phenyl, Naphthyl, Biphenyl, Triazinyl, 2,4-Diphenyl-1,3,5-triazin-6-yl-phenyl, Benzoylphenyl, Benzylphenyl, α-Methylbenzylphenyl, α,α-Dimethylbenzylphenyl wie Methylphenyl, Dimethylphenyl, Trimethylphenyl, Ethylphenyl, Vinylphenyl, Diethylphenyl, Isopropylphenyl, tert.-Butylphenyl, Di-tert-butylphenyl, Methyl-di-t-butylphenyl, 1,1,3,3-Tetramethylbutylphenyl und 1,1,3,3,5,5-Hexamethylhexylphenyl, Dodecenylphenyl, 1-Methylnaphthyl, 2-Methylnaphthyl, 1-Ethylnaphthyl, 2-Propylbiphenyl-4-yl, 4-(1-Hex-3-enyl)-biphenyl-8-yl; bevorzugt Phenyl, das gegebenenfalls mit 1-3, z.B. 1-2, insbesondere 1 $C_1$-$C_4$-Alkylgruppe(n), vor allem Methyl, substituiert ist.

Die divalenten (für m = 2 bzw. n = 2), trivalenten (für m = 3 bzw. n = 3), tetravalenten (für m = 4 bzw. n = 4), pentavalenten (für m = 5 bzw. n = 5) und hexavalenten Reste $A^1$ und $A^2$ (für m = 6 bzw. n = 6) leiten sich von den oben genannten monovalenten Resten ab. Ein divalenter Rest unterscheidet sich von dem entsprechenden monovalenten Rest dadurch, daß er an Stelle eines Wasserstoffatoms eine offene Bindung enthält; ein trivalenter Rest unterscheidet sich von dem entsprechenden monovalenten Rest dadurch, daß er an Stelle zweier Wasserstoffatome zwei offene Bindungen enthält; ein tetravalenter Rest unterscheidet sich von dem entsprechenden monovalenten Rest dadurch, daß er an Stelle dreier Wasserstoffatome drei offene Bindungen enthält; ein pentavalenter Rest unterscheidet sich von dem entsprechenden monovalenten Rest dadurch, daß er an Stelle von vier Wasserstoffatomen vier offene Bindungen enthält; ein hexavalenter Rest unterscheidet sich von dem entsprechenden monovalenten Rest dadurch, daß er an Stelle von fünf Wasserstoffatomen fünf offene Bindungen enthält. So kann $A^1$ im Rahmen der angegebenen Bedeutungen z.B. als divalenter Rest auch Methylen, Ethylen, -$CH_2$-C($CH_3$)$_2$-$CH_2$-, Prop-2-en-yliden, Propylen(1,3), Butylen(1,4), Pentylen(1,5), Hexylen(1,6),

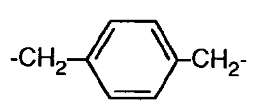 , 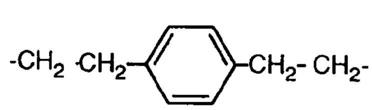 ,

-$CH_2$-S-$CH_2$-, -$C_2H_4$-S-$C_2H_4$- oder -$C_2H_4$-O-$C_2H_4$-, Cyclohexylen; als trivalenter Rest beispielsweise

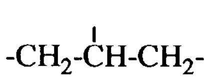 , 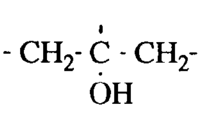 , N(-$CH_2$-)$_3$, 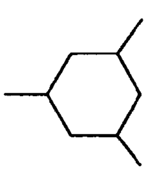 oder 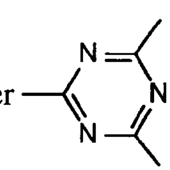 ;

als tetravalenter Rest beispielsweise

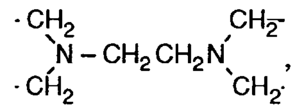 , 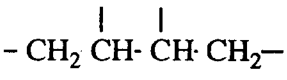

oder

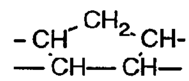

darstellen;
gemeinsame Bedeutungsmöglichkeiten für $A^1$ und $A^2$ als divalente Reste sind beispielsweise o-, m- oder p-Phenylen, $-C_6H_4-C(CH_3)_2-C_6H_4-$, $-C_6H_4-CO-C_6H_4-$; als trivalente Reste beispielsweise

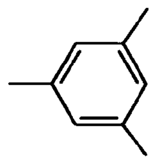 , 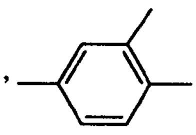 oder 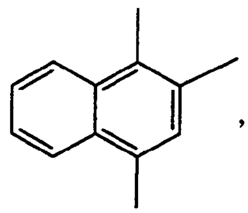 ,

als tetravalenter Rest z.B.

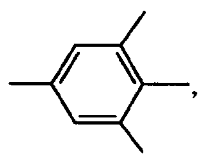 , 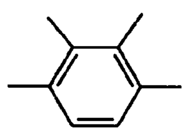 oder 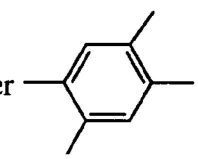

darstellen;

$A^2$ kann sich beispielsweise von einem n-wertigen Phenol der Formel

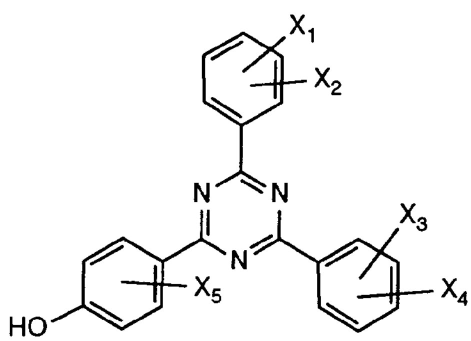

ableiten, wobei $X_1$ bis $X_5$, unabhängig voneinander, Wasserstoff, Methyl oder OH bedeuten können.

Wie oben beschrieben, leitet sich die Gruppe $[-O-CO]_m-A^1$ in Formel I von einer m-basischen Carbonsäure ab. Es kann sich dabei um aromatische, aliphatische oder gemischt aromatisch-aliphatische Säure handeln, wobei cycloaliphatische und bicycloaliphatische Säuren und/oder ungesättigte Säuren ausdrücklich eingeschlossen werden. Es kann sich beispielsweise um folgende Säuren handeln:
Caprylsäure, Essigsäure, Stearinsäure, Polyisobutenylbernsteinsäure, n-Hexacosansäure, Trimethylessigsäure, Propionsäure, Isovaleriansäure, Laurinsäure, Ölsäure, Acrylsäure, Methacrylsäure, Sorbinsäure, Linolensäure, Maleinsäure, Itaconsäure, Glutaconsäure, dibasische Säuren wie Oxal-, Malon-, Dibutylmalon-, Dibenzylmalon-, Bernstein-, Iso-dodecylbernstein-, Glutar-, Adipin-, Pimelin-, Kork-, Azelain- oder Sebacinsäure, die Polymerisate von Fettsäuren, wie die Dimeren und Trimeren derselben vom Typ, wie sie

beispielsweise in Ind. and Eng. Chem. 33, 86-89 (1941) beschrieben sind, Butan-1,2,3,4-tetracarbonsäure, Heteroatome enthaltende Säuren, wie beispielsweise Thiodiglycolsäure, Citronensäure, Nitrilotriessigsäure, Ethylendiamintetraessigsäure, 1,3,5-Triazin-2,4,6-tricarbonsäure, cycloaliphatische Säuren, wie Cyclohexancarbonsäure, 1,2- und 1,4-Cyclohexandicarbonsäure und Naphthensäuren, darunter z.B. Cyclopentancarbonsäure, Cyclopentan-1,2,3,4-tetracarbonsäure, Cyclopentylessigsäure, 3-Methylcyclopentylessigsäure, Camphersäure, 4-Methylcyclohexancarbonsäure und 2,4,6-Trimethylcyclohexancarbonsäure, Bicyclo-(2,2,2)-octa-5-en-2,3-dicarbonsäure und Bicyclo-(2,2,1)-hepta-5-en-2-carbonsäure, aromatische Carbonsäuren, wie Benzoesäure, Benzoylbenzoesäure, o-, m- und p-Toluylsäure, Phthalsäure, Terephthalsäure, Trimellitsäure, Trimesinsäure, Pyromellitsäure, 1,2,4,5-Benzoltetracarbonsäure, Diphensäure, 1-Naphthoesäure, 2-Naphthoesäure, Naphthalin-1,8-dicarbonsäure, Naphthalin-1,4-dicarbonsäure, Naphthalin-1,4,5-tricarbonsäure usw., paraffinische ω-Arylsäuren, wie Phenylessigsäure, Hydrozimtsäure, Phenylbuttersäure, γ-(1-Naphthyl)-buttersäure, δ-Phenylen-n-valeriansäure, ε-Phenyl-n-capronsäure, o-, m- oder p-Phenylendiessigsäure oder o-Phenylenessig-β-propionsäure, sowie ungesättigte Phenylsäuren wie beispielsweise Zimtsäure.

$R^1$ umfaßt beispielsweise die folgenden Bedeutungen:

Verzweigtes oder unverzweigtes $C_1$-$C_{36}$-Alkyl wie Methyl, Ethyl, Propyl, Isopropyl, n-Butyl, sec-Butyl, Isobutyl, t-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl, Docosyl, Pentakosyl oder Triakosyl; bevorzugt ist unverzweigtes Alkyl, besonders bevorzugt unverzweigtes $C_1$-$C_{18}$-Alkyl; verzweigtes oder unverzweigtes $C_1$-$C_{36}$-Alkyloxy, insbesondere $C_6$-$C_{18}$-Alkyloxy wie Hexyloxy, Heptyloxy, Octyloxy, Nonyloxy, Decyloxy, Undecyloxy, Dodecyloxy;

$C_3$-$C_{36}$-Alkenyl wie Propenyl (Allyl), Butenyl, Pentenyl, Hexenyl, Heptenyl, Octenyl, Nonenyl, Decenyl, Dodecenyl, Pentadecenyl, Octadecenyl; $C_3$-$C_{36}$-Alkenyloxy;

$C_3$-$C_9$-Alkinyl, z.B. Propargyl;

durch $C_5$-$C_8$-Cycloalkyl substituiertes Alkyl oder Alkyloxy wie Cyclopentylmethyl, Cyclohexylmethyl, Cycloheptylmethyl, Cyclooctylmethyl, Cyclohexylethyl, 2-Cyclohexyl-n-propyl, 3-Cyclohexyl-n-propyl, 4-Cyclohexyl-n-butyl; durch $C_5$-$C_8$-Cycloalkyl oder 1 bis 6 -O- unterbrochenes Alkyl oder Alkyloxy wie beispielsweise der Formeln

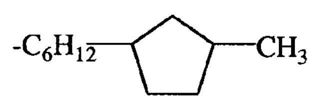 ,

$-C_2H_4$-O-$C_2H_4$-O-$C_{12}H_{25}$, $-(C_2H_4\text{-}O)_4$-$C_4H_9$, $-(C_2H_4\text{-}O)_6$-$C_4H_9$;

$C_5$-$C_8$-Cycloalkyl, $C_5$-$C_8$-Cycloalkyloxy, $C_6$-$C_8$-Cycloalkenyl oder $C_6$-$C_8$-Cycloalkenyloxy, das unsubstituiert oder mit $C_1$-$C_{12}$-Alkyl oder $C_2$-$C_{12}$-Alkenyl substituiert ist, wie Cyclopentyl, Cyclopentyloxy, Cyclohexyl, Cyclohexyloxy, Cycloheptyl, Cycloheptyloxy, Cyclooctyl, Cyclooctyloxy, 2- oder 4-Methylcyclohexyloxy, Dimethylcyclohexyloxy, Trimethylcyclohexyl, t-Butylcyclohexyl, 2-Cyclohexenyl, besonders Cyclohexyl und Cyclohexyloxy;

Phenyl, Phenoxy, Benzylphenyl, Benzylphenyloxy;

mit $C_1$-$C_{12}$-Alkyl oder $C_2$-$C_{12}$-Alkenyl substituiertes Phenyl oder Phenyloxy;

$C_7$-$C_{12}$-Phenylalkyl, $C_7$-$C_{12}$-Phenylalkenyl, $C_7$-$C_{12}$-Phenylalkyloxy, $C_7$-$C_{12}$-Phenylalkenyloxy, wie z.B. Benzyl, Benzyloxy, Phenethyloxy, 3-Phenylpropyloxy, α-Methylbenzyl, α-Methylbenzyloxy, α,α-Dimethylbenzyl, α,α-Dimethylbenzyloxy.

Bevorzugt sind Verbindungen der Formeln I und II, worin m und n, unabhängig voneinander, eine Zahl aus dem Bereich von 1 bis 4 sind, und insbesondere solche, worin m eine Zahl aus dem Bereich von 1 bis 4 und n eine Zahl aus dem Bereich von 1 bis 3 ist. Von besonderem Interesse sind solche Verbindungen der Formeln I und II, in denen m und n 1 oder 2, vor allem 2 sind.

Bevorzugt sind Verbindungen der Formel I und II, worin

m und n 1, 2, 3 oder 4 sind;

$A^1$ im Fall m = 1 $C_1$-$C_{25}$-Alkyl darstellt, das unsubstituiert oder durch $C_5$-$C_8$-Cycloalkyl substituiert ist; oder $C_2$-$C_{25}$-Alkyl darstellt, das durch $C_5$-$C_8$-Cycloalkyl, -S-, -O- oder $-NR^2$- oder verschiedener dieser Gruppen unterbrochen ist; oder $A^1$ im Fall m = 1 $C_5$-$C_8$-Cycloalkyl, das unsubstituiert oder mit $C_1$-$C_{12}$-Alkyl substituiert ist; $C_6$-$C_8$-Cycloalkenyl, das unsubstituiert oder mit $C_1$-$C_{12}$-Alkyl substituiert ist; Phenyl, das unsubstituiert oder durch 1 bis 3 $C_1$-$C_4$-Alkyl, $C_2$-$C_4$-Alkenyl- oder $C_1$-$C_4$-Alkoxyreste und/oder eine Gruppe der Formel

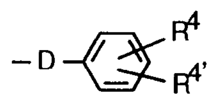

substituiert ist; oder $C_2$-$C_{25}$-Alkenyl; $C_3$-$C_{25}$-Alkenyl, das durch -O- unterbrochen ist; $C_7$-$C_{12}$-Phenylalkyl, das unsubstituiert oder durch $C_1$-$C_4$-Alkyl oder $C_2$-$C_4$-Alkenyl oder $C_1$-$C_4$-Alkoxy substituiert ist, bedeutet;

$A^1$ im Fall m = 2 eine direkte Bindung; $C_1$-$C_{18}$-Alkylen; $C_2$-$C_{18}$-Alkenylen; $C_5$-$C_8$-Cycloalkylen, das unsubstituiert oder mit $C_1$-$C_{12}$-Alkyl substituiert ist; $C_6$-$C_8$-Cycloalkenylen, das unsubstituiert oder mit $C_1$-$C_{12}$-Alkyl substituiert ist; Phenylen, das unsubstituiert oder durch 1 oder 2 $C_1$-$C_4$-Alkyl-, $C_2$-$C_4$-Alkenyl- oder $C_1$-$C_4$-Alkoxyreste und/oder eine Gruppe der Formel

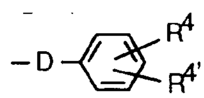

substituiert ist; einen zweiwertigen Rest der Formel

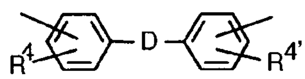

;

einen zweiwertigen heterozyklischen Rest aus der Gruppe Furan, Thiophen oder Pyrrol, welches am Stickstoffatom durch Wasserstoff oder den Substituenten $-R^2$ abgesättigt ist; oder $A^1$, im Fall m = 2, durch $C_5$-$C_8$-Cycloalkylen, Phenylen, -S-, -O- oder $-NR^2-$ oder verschiedener dieser Gruppen unterbrochenes $C_2$-$C_{18}$-Alkylen bedeutet; und

$A^1$ im Fall m=3 $C_1$-$C_8$-Alkantriyl; $C_2$-$C_8$-Alkentriyl; Benzoltriyl; einen trivalent Rest der Formel

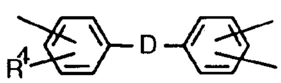 oder 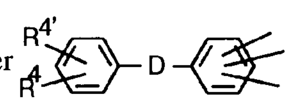 ;

eine trivalente Gruppe der Formel

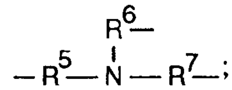

;

1,3,5-Triazin-2,4,6-triyl; $C_5$-$C_8$-Cycloalkantriyl; oder $C_2$-$C_{18}$-Alkantriyl, das durch -S-, -O- oder $-NR^2-$ unterbrochen ist und/oder durch tertiär -OH substituiert ist, bedeutet;

$A^1$ im Fall m = 4 einen Benzol-, Cyclopentyl- oder Cyclohexylrest mit 4 freien Valenzen oder einen tetravalenten Rest der Formel

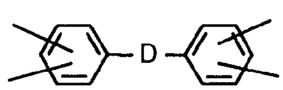

,

einen tetravalenten Rest der Formel

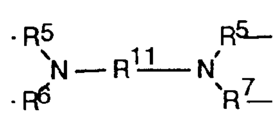

,

oder $C_1$-$C_8$-Alkantetrayl bedeutet;

$A^2$ im Fall n = 1 Phenvl. das unsubstituiert oder durch 1 bis 3 $C_1$-$C_4$-Alkyl-, $C_2$-$C_4$-Alkenyl- oder $C_1$-$C_4$-Alkoxy-

reste und/oder eine Gruppe der Formel

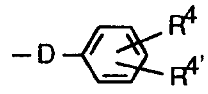

substituiert ist; einen Triazinylphenylrest der Formel

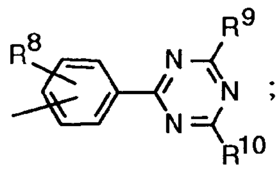 ;

oder Naphthyl bedeutet;
$A^2$ im Fall n = 2 Phenylen, das unsubstituiert oder durch 1 oder 2 $C_1$-$C_4$-Alkyl-, $C_2$-$C_4$-Alkenyl- oder $C_1$-$C_4$-Alkoxyreste und/oder eine Gruppe der Formel

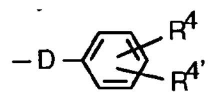

substituiert ist; einen zweiwertigen Rest der Formel

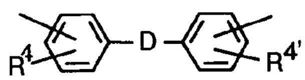 ;

oder Naphthylen;
$A^2$ im Fall n = 3 Benzoltriyl; oder einen trivalenten Rest der Formel

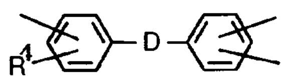 oder 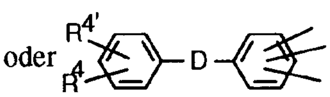 ;

$A^2$ im Fall n = 4 einen Benzolrest mit 4 freien Valenzen oder einen tetravalenten Rest der Formel

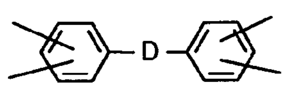

bedeutet;
oder $A^2$ einen n-wertigen Triphenyltriazinylrest der Formel

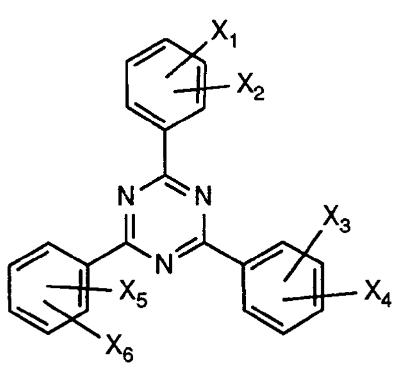

darstellt, wobei 1 bis 4 der Reste $X_1$ bis $X_6$ jeweils eine offene Bindung darstellen und die übrigen, unabhängig voneinander, Wasserstoff oder $C_1$-$C_4$-Alkyl bedeuten;
D eine direkte Bindung; $C_1$-$C_{10}$-Alkylen; oder eine der Gruppen -CO-, -S-, -O- oder -$SO_2$- darstellt;
$R^1$ die Bedeutung $C_1$-$C_{36}$-Alkyl; $C_3$-$C_{36}$-Alkenyl; $C_3$-$C_9$-Alkinyl; $C_1$-$C_{36}$-Alkoxy; $C_3$-$C_{36}$-Alkenyloxy; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist; $C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; Phenyl, welches unsubstituiert oder durch 1 bis 3 $C_1$-$C_4$-Alkyl- oder $C_1$-$C_4$-Alkoxyreste substituiert ist; Naphthyl, welches unsubstituiert oder durch 1 bis 3 $C_1$-$C_4$-Alkyl- oder $C_1$-$C_4$-Alkoxyreste substituiert ist; Phenoxy, welches unsubstituiert oder durch 1 bis 3 $C_1$-$C_4$-Alkyl- oder $C_1$-$C_4$-Alkoxyreste substituiert ist; Naphthoxy, welches unsubstituiert oder durch 1 bis 3 $C_1$-$C_4$-Alkyl- oder $C_1$-$C_4$-Alkoxyreste substituiert ist;
$C_7$-$C_{12}$-Phenylalkyl, welches unsubstituiert oder am Phenylring durch 1 bis 3 $C_1$-$C_4$-Alkyl- oder $C_1$-$C_4$-Alkoxyreste substituiert ist; $C_7$-$C_{12}$-Phenylalkoxy, welches unsubstituiert oder am Phenylring durch 1 bis 3 $C_1$-$C_4$-Alkyl- oder $C_1$-$C_4$-Alkoxyreste substituiert ist; oder -CO-$R^2$ hat; wobei
$R^2$ $C_1$-$C_{18}$-Alkyl; $C_5$-$C_8$-Cycloalkyl; Phenyl; Naphthyl; $C_7$-$C_9$-Phenylalkyl; oder $C_{11}$-$C_{14}$-Naphthylalkyl bedeutet; und
$R^3$ dieselben Bedeutungen umfaßt wie $R^2$ oder Wasserstoff bedeutet;
$R^4$ und $R^{4'}$, unabhängig voneinander, Wasserstoff oder $C_1$-$C_4$-Alkyl;
$R^5$, $R^6$ und $R^7$ unabhängig voneinander $C_1$-$C_3$-Alkylen;
$R^8$ Wasserstoff oder $C_1$-$C_4$-Alkyl;
$R^9$ und $R^{10}$, unabhängig voneinander, $C_3$-$C_{18}$-Alkyl oder $C_5$-$C_8$-Cycloalkyl; und
$R^{11}$ $C_2$-$C_6$-Alkylen darstellen.

In den obigen Formeln vom Typ

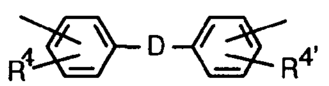

stellen die in den Phenylkern hineinragenden Striche mit dem Symbol $R^4$ bzw. $R^{4'}$ Substituenten dar, die sich in o-, m- oder p-Position zu dem Brückenglied D befinden; die Striche ohne Symbole bezeichnen offene Bindungen an anderen, noch freien Positionen in o-, m- oder p-Position zu D.

D ist vorzugsweise eine der Gruppen -CO-, -$CH_2$-, -O- oder

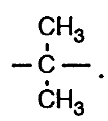

$R^4$ und $R^{4'}$ bedeuten vorzugsweise Wasserstoff, Methyl oder tert.-Butyl; besonders Wasserstoff.

$R^1$ hat besonders bevorzugt die Bedeutung $C_1$-$C_6$-Alkyl, $C_6$-$C_{12}$-Alkoxy, Cyclohexyl, Cyclohexyloxy, Cycloheptyloxy, Cyclooctyloxy, $C_7$-$C_9$-Phenylalkyl oder $C_7$-$C_9$-Phenylalkoxy; vor allem Methyl, Cyclohexyloxy, Benzyl, Benzyloxy, α-Methylbenzyl, α-Methylbenzyloxy, Octyloxy und Dodecyloxy, insbesondere Methyl, Cyclohexyloxy, Benzyl und Octyloxy.

Von besonders herausragendem Interesse sind Verbindungen der Formel I, worin $A^1$ m- oder p-Phenylen; 1,3,5-Benzoltriyl; Ethylen; 1,4-Cyclohexylen; n-Heptadecyl; n-Undecyl; 1,6-Hexylen; 1,8-Octylen; 1,10-Decylen oder 1,12-Dodecylen; vor allem m-Phenylen; Ethylen; 1,8-Octylen; 1,10-Decylen; 1,4-Cyclohexylen oder n-Undecyl ist, sowie Verbindungen der Formel II, worin $A^2$ m-Phenylen oder eine divalenter Rest der Formel

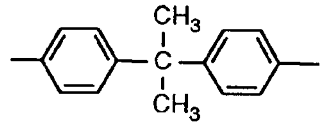

ist.

Weiterhin hervorzuheben sind Verbindungen der Formeln I und II, worin $A^1$ im Fall m = 1 $C_6$-$C_{18}$-Alkyl; $C_2$-$C_{12}$-Alkenyl; Cyclohexyl; Cyclohexenyl; Phenyl, das unsubstituiert oder durch 1 bis 3 Reste $C_1$-$C_4$-Alkyl oder

eine Gruppe der Formel

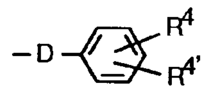

oder $C_2$-$C_4$-Alkenyl substituiert ist; $C_3$-$C_{12}$-Alkenyl, das durch -O- unterbrochen ist; oder $C_7$-$C_{12}$-Phenylalkyl, das unsubstituiert oder am Phenylring durch $C_1$-$C_4$-Alkyl oder $C_2$-$C_4$-Alkenyl oder $C_1$-$C_4$-Alkoxy substituiert ist, darstellt;

$A^1$ im Fall m = 2 eine direkte Bindung; $C_1$-$C_{18}$-Alkylen; $C_2$-$C_{18}$-Alkylen, das durch -O- oder -S- unterbrochen ist; $C_2$-$C_{18}$-Alkenylen; $C_5$-$C_9$-Cycloalkylen; Phenylen, das unsubstituiert oder durch 1 oder 2 Reste $C_1$-$C_4$-Alkyl oder $C_1$-$C_4$-Alkoxy und/oder eine Gruppe der Formel

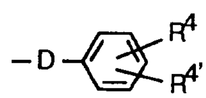

oder $C_2$-$C_4$-Alkenyl substituiert ist; einen zweiwertigen Rest der Formel

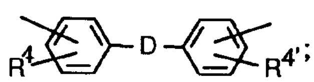

$A^1$ im Fall m = 3 $C_3$-$C_8$-Alkantriyl; durch eine tertiäre Hydroxylgruppe substituiertes $C_3$-$C_8$-Alkantriyl; Benzoltriyl; $N[(CH_2)-]_3$; $C_5$-$C_8$-Cycloalkantriyl; oder 1,3,5-Triazin-2,4,6-triyl;

$A^1$ im Fall m = 4 einen Benzol-, Cyclopentyl- oder Cyclohexylrest mit 4 freien Valenzen; oder

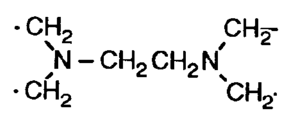

bedeutet;

$A^2$ im Fall n = 1 Phenyl, das unsubstituiert oder durch $C_1$-$C_4$-Alkyl oder eine Gruppe der Formel

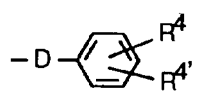

oder $C_2$-$C_4$-Alkenyl substituiert ist; oder Naphthyl bedeutet;

$A^2$ im Fall n = 2 Phenylen, das unsubstituiert oder durch $C_1$-$C_4$-Alkyl oder eine Gruppe der Formel

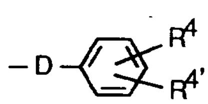

substituiert ist; einen zweiwertigen Rest der Formel

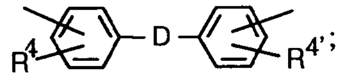

oder Naphthylen;

$A^2$ im Fall n = 3 Benzoltriyl;

$A^2$ im Fall n = 4 einen Benzolrest mit 4 freien Valenzen bedeutet; oder $A^2$ einen n-wertigen Triphenyltriazinylrest der Formel

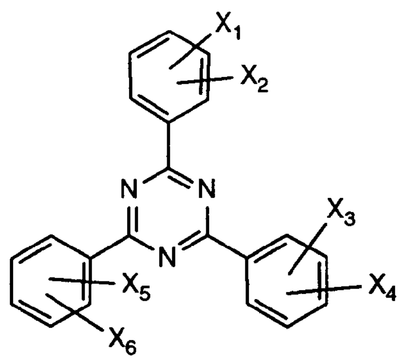

darstellt, wobei 1 bis 4 der Reste $X_1$ bis $X_6$ jeweils eine offene Bindung darstellen und die übrigen, unabhängig voneinander, Wasserstoff oder Methyl bedeuten;
D eine direkte Bindung oder eine der Gruppen -CO-, -S-, -O-, $-SO_2-$ oder

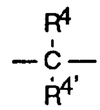

darstellt;
$R^1$ die Bedeutung $C_1$-$C_{18}$-Alkyl; $C_4$-$C_{22}$-Alkoxy; $C_3$-$C_8$-Alkenyl; $C_3$-$C_8$-Alkinyl; $C_5$-$C_8$-Cycloalkyl; $C_5$-$C_8$-Cycloalkoxy; $C_7$-$C_{12}$-Phenylalkyl; $C_7$-$C_{12}$-Phenylalkoxy; $C_1$-$C_8$-Alkanoyl; $C_3$-$C_5$-Alkenoyl; oder Benzoyl hat; und
$R^4$ und $R^{4'}$, unabhängig voneinander, Wasserstoff oder $C_1$-$C_4$-Alkyl darstellen.

Von besonderem Interesse sind Verbindungen der Formeln I und II, worin m und n jeweils ganze Zahlen aus dem Bereich 1 bis 4 sind;
$A^1$ im Fall m = 1 $C_6$-$C_{18}$-Alkyl;
$A^1$ im Fall m = 2 $C_2$-$C_{18}$-Alkylen; Cyclohexylen; Phenylen; einen zweiwertigen Rest der Formel

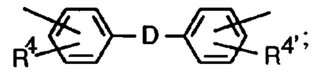;

$A^1$ im Fall m = 3 Benzoltriyl oder Cyclohexantriyl; und
$A^1$ im Fall m = 4 einen Benzolrest mit 4 freien Valenzen bedeutet;
$A^2$ im Fall n = 1 Phenyl, das unsubstituiert oder durch $C_1$-$C_4$-Alkyl substituiert ist; oder Naphthyl bedeutet;
$A^2$ im Fall n = 2 Phenylen, das unsubstituiert oder durch $C_1$-$C_4$-Alkyl oder eine Gruppe der Formel

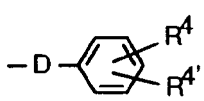

substituiert ist; Naphthylen; oder einen zweiwertigen Rest der Formel

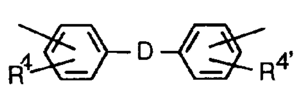

bedeutet;
$A^2$ im Fall n = 3 Benzoltriyl; und
$A^2$ im Fall n = 4 einen Benzolrest mit 4 freien Valenzen darstellt; D eine der Gruppen $-CH_2-$, -CO- oder

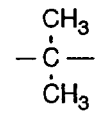

darstellt; und
$R^4$ und $R^{4'}$, unabhängig voneinander, Wasserstoff, Methyl oder tert.-Butyl darstellen.

Besonders bevorzugt sind Verbindungen der Formeln I und II, worin m eine ganze Zahl aus dem Bereich 1 bis 4 und n eine ganze Zahl aus dem Bereich 1 bis 3 ist;
$A^1$ im Fall m = 1 $C_{10}$-$C_{18}$-Alkyl;
$A^1$ im Fall m = 2 $C_2$-$C_{18}$-Alkylen; Cyclohexylen; oder Phenylen;
$A^1$ im Fall m = 3 Benzoltriyl oder Cyclohexantriyl bedeutet; und
$A^1$ im Fall m = 4 Benzoltetrayl ist;
$A^2$ im Fall n = 1 Phenyl;
$A^2$ im Fall n = 2 Phenylen oder einen zweiwertigen Rest der Formel

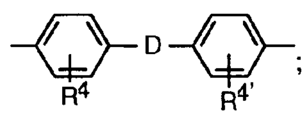

und $A^2$ im Fall n = 3 Benzoltriyl bedeutet:
D eine der Gruppen -CO- oder

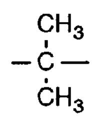

darstellt;
$R^1$ die Bedeutung $C_1$-$C_6$-Alkyl; $C_6$-$C_{12}$-Alkoxy; Cyclohexyl; Cyclohexyloxy; Cycloheptyloxy; Cyclooctyloxy; oder $C_7$-$C_9$-Phenylalkyl hat; und
$R^4$ und $R^{4'}$, unabhängig voneinander, Wasserstoff, Methyl oder tert.-Butyl darstellen.

Von besonderer Bedeutung sind Verbindungen der Formel I oder der Formel II, die sich von einer im aliphatischen Teil ethylenisch ungesättigten Carbonsäure oder einem im aliphatischen Teil ethylenisch ungesättigten Phenolderivat ableiten. Solche Verbindungen weisen einerseits die beschriebene stabilisierende Wirkung auf, sie lassen sich andererseits auch durch Polymerisation zu hochmolekularen Stabilisatoren umsetzen und stellen damit wertvolle Zwischenprodukte zur Herstellung polymerer Lichtschutzmittel dar.

Beispiele für solche Verbindungen sind Verbindungen der Formeln I und II, worin m 1 oder 2 und n 1 ist;
$A^1$ im Fall m = 1 $C_2$-$C_{12}$-Alkenyl; Cyclohexylen; Phenyl, das durch $C_2$-$C_4$-Alkenyl und optional durch $C_1$-$C_4$-Alkyl oder eine Gruppe der Formel

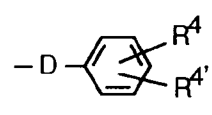

substituiert ist;
$C_3$-$C_{12}$-Alkenyl, das durch -O- unterbrochen ist; oder $C_7$-$C_{12}$-Phenylalkyl, das am Phenylring durch $C_2$-$C_4$-Alkenyl substituiert ist;
$A^1$ im Fall m = 2 $C_2$-$C_{18}$-Alkenylen; oder Phenylen, das am Phenylring durch $C_2$-$C_4$-Alkenyl substituiert ist;
$A^2$ im Fall n = 1 Phenyl, das durch $C_2$-$C_4$-Alkenyl substituiert ist;
D eine direkte Bindung oder eine der Gruppen -O- oder

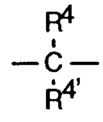

darstellt;
$R^1$ die Bedeutung $C_1$-$C_{18}$-Alkyl; $C_4$-$C_{22}$-Alkoxy; $C_5$-$C_8$-Cycloalkyl; $C_5$-$C_8$-Cycloalkoxy; $C_7$-$C_{12}$-Phenylalkyl; $C_1$-$C_8$-Alkanoyl; oder Benzoyl hat; und
$R^4$ und $R^{4'}$, unabhängig voneinander, Wasserstoff oder $C_1$-$C_4$-Alkyl darstellen.

Unter den genannten Derivaten ethylenisch ungesättigter Carbonsäuren und Alkenylphenole lassen sich besonders vorteilhaft diejenigen einsetzen, in denen m und n jeweils 1 sind, und darunter vor allem solche, worin
$A^1$ Vinyl; α-Methylvinyl; durch Vinyloxy substituiertes $C_1$-$C_4$-Alkyl; Vinylphenyl oder Vinylbenzyl; und
$A^2$ Vinylphenyl darstellt.

Ein bevorzugtes Verfahren zur Herstellung einer Verbindung der Formel I oder der Formel II, welches ebenfalls einen Gegenstand der Erfindung bildet, geht von einer Piperidinverbindung der Formel III aus:

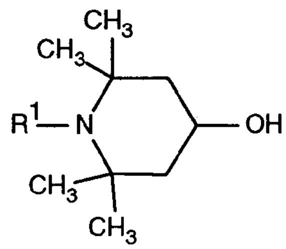

(III)

Verbindungen der Formel III sind bekannt und zum Teil kommerziell erhältlich.

Die Piperidinverbindung der Formel III wird zunächst in an sich bekannter Weise unter Abspaltung von HCl mit Epichlorhydrin zum Zwischenprodukt der Formel IV

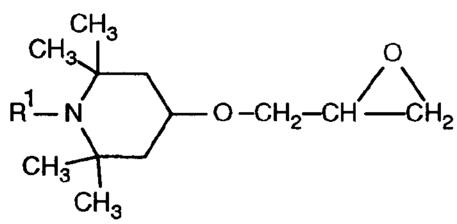

(IV)

umgesetzt und dieses anschließend
(a) mit einer Carbonsäure der Formel V

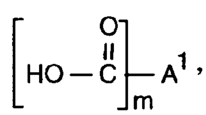

(V)

unter Bildung des Esters der Formel I, oder
(b) mit einem Phenol der Formel VI

$$[HO]_n-A^2 \qquad (VI)$$

unter Bildung des Phenolethers der Formel II zur Reaktion gebracht. Die Symbole m, n, $R^1$, $A^1$ und $A^2$ haben dabei die weiter oben angegebenen Bedeutungen.

Die Herstellung des Zwischenproduktes der Formel IV (Epoxid) kann gemäß einer der Methoden erfolgen, die in der EP-A-001835 oder bei Luston und Vass, Makromol. Chem., Macromol. Symp. 27, 231 (1989), beschrieben sind. Zweckmäßig wird die Piperidinverbindung der Formel III in Gegenwart starker Basen, beispielsweise wässriger konzentrierter Alkalihydroxidlösung, und gegebenenfalls eines organischen Lösemittels langsam mit einem Überschuß Epichlorhydrin versetzt.

Vorteilhaft wird die Base in ca. 2-20-fachem molarem Überschuß bezogen auf die Verbindung der Formel

III eingesetzt; beispielsweise werden 3-15 Mol, bevorzugt 4-12 Mol Natrium- oder Kaliumhydroxid als 50-% wässrige Lösung pro Mol Piperidinverbindung verwendet. Die Menge des organischen Lösemittels wird zweckmäßig so bemessen, daß die Verbindung der Formel III vollständig gelöst wird; als Lösemittel eignen sich beispielsweise inerte Lösemittel wie Kohlenwasserstoffe oder Ether; bevorzugt ist Toluol.

Auf ein Äquivalent der Piperidinverbindung der Formel III können beispielsweise 1-4, vorzugsweise 1,2-3, vor allem 1,5-2,5 Äquivalente Epichlorhydrin eingesetzt werden. Daruberhinaus können der Mischung vorteilhaft 1-30 Mol-%, bevorzugt 5-25 Mol-%, eines tert. Aminsalzes, beispielsweise eines Tetraalkylammoniumhalogenids wie Tetramethylammoniumchlorid oder Tetrabutylammoniumbromid, oder eines Phosphoniumsalzes, beispielsweise eines quaternären Phosphoniumhalogenids wie Ethyltriphenylphosphoniumbromid, als Katalysator zugesetzt werden.

Die Temperatur beträgt während der Reaktion zweckmäßig 0-100°C, vorzugsweise 20-80°C, vor allem 30-70°C.

Nach vollständiger Reaktion kann die Aufarbeitung nach gängigen Methoden erfolgen; zweckmäßig wird die Mischung zunächst mit Wasser verdünnt, beispielsweise indem das Reaktionsgemisch in das 1-4-fache Volumen Eiswasser gegeben wird; anschließend kann die organische Phase direkt abgetrennt oder extrahiert werden, zur Extraktion eignet sich z.B. Essigester. Das Produkt kann nach Trocknen der organischen Phase durch Entfernen des Lösemittels isoliert werden. Möglich ist auch das Einschalten weiterer Reinigungsschritte wie Dispergieren von Aktivkohle, Filtrieren oder Destillieren.

Die weitere Umsetzung des Zwischenproduktes der Formel IV unter Öffnen des Epoxides zur Verbindung der Formel I oder II kann mit oder ohne Lösemittel durchgeführt werden; gegebenenfalls einsetzbare Lösemittel sind polar oder unpolar, inert und, im Falle der Formel II, bevorzugt hochsiedend. So können beispielsweise aromatische oder aliphatische Kohlenwasserstoffe ebenso verwendet werden wie heterozyklische Lösemittel, Ether, Sulfone, Sulfoxide oder Amide; bevorzugte Lösemittel sind unter anderem höhersiedende Kohlenwasserstofffraktionen wie Ligroin oder Petrolether, aromatische Kohlenwasserstoffe wie Toluol oder Xylol, Dekalin, cyclische oder offenkettige Ether wie Dibutylether oder Dioxan, Dimethylformamid, Dimethylsulfoxid; besonders bevorzugt sind Toluol oder Xylol.

Die Temperatur der Reaktionsmischung kann für die Dauer der Reaktion im Siedebereich gehalten werden (Rückfluß). Dazu wird eine Lösemittel enthaltende Reaktionsmischung, im Allgemeinen unter Normaldruck, zum Siedepunkt erwärmt und das verdampfte Lösemittel mit Hilfe eines geeigneten Kühlers kondensiert und wieder der Reaktionsmischung zugeführt. Der Siedebereich des reinen Lösemittels kann gegebenenfalls im Bereich von 60-180°C liegen, beispielsweise 60-140°C. Wird zur Umsetzung eine Carbonsäure der Formel V eingesetzt, wird die Temperatur bevorzugt im Bereich 30-130°C, vor allem 40-90°C gehalten, die Reaktionsdauer beträgt beispielsweise 1 bis 20 Stunden. Im Falle des Einsatzes eines Phenols der Formel VI wird die Temperatur bevorzugt im Bereich 80-180°C, vor allem 100-160°C gehalten, die Reaktionsdauer beträgt beispielsweise 3 bis 36 Stunden.

Die Reaktion wird vorzugsweise unter Schutzgas, z.B. Stickstoff oder Argon, durchgeführt; die Reaktionsmischung wird zweckmäßig gerührt. Das Epoxid der Formel IV wird vorzugsweise in etwa äquivalenter Menge oder in leichtem Überschuß bezogen auf die Carbonsäuregruppen des Reaktanden der Formel V oder die phenolischen Hydroxylgruppen des Reaktanden der Formel VI eingesetzt, beispielsweise in einer Menge von 1,0 bis 1,3 Äquivalente, vor allem 1,0 bis 1,15 Äquivalente pro Äquivalent phenolisches OH oder Carbonsäure.

Die Reaktion wird vorzugsweise in Anwesenheit eines quaternären Ammoniumsalzes, beispielsweise eines Tetraalkylammoniumhalogenids wie z.B. Tetramethylammoniumchlorid oder Tetrabutylammoniumbromid, oder eines Phosphoniumsalzes, beispielsweise eines quaternären Phosphoniumhalogenids wie Ethyltriphenylphosphoniumbromid, als Katalysator durchgeführt. Der Katalysator wird zweckmäßig in einer Menge von 1 bis 5 Mol-% bezogen auf die Verbindung der Formel IV eingesetzt.

Die Aufarbeitung nach Beendigung der Reaktion kann nach gängigen Methoden erfolgen; beispielsweise wird die erkaltete Mischung zunächst mit der wässrigen Lösung einer Base, z.B. stark verdünnter Alkalilauge, und anschließend mit Wasser gewaschen und nach Trocknen das Lösemittel entfernt, z.B. durch Anlegen von Unterdruck und/oder Erwärmen. Nach dem Trocknen können noch weitere Reinigungsschritte wie Dispergieren von Aktivkohle, Filtrieren, Destillieren usw. eingeschaltet werden.

In einem weiteren möglichen Herstellungsverfahren für die erfindungsgemäßen Verbindungen wird Epichlorhydrin zunächst mit der Carbonsäure der Formel V beziehungsweise mit dem Phenol der Formel VI umgesetzt, und das erhaltene Zwischenprodukt anschließend unter Öffnen des Epoxidringes mit der Piperidinverbindung der Formel III zum gewünschten Ester der Formel I beziehungsweise Phenolether der Formel II reagiert.

Die Verbindungen der Formeln I und II eignen sich zum Stabilisieren von organischen Materialien gegen thermischen, oxidativen und aktinischen Abbau.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze .

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Weitere Gegenstände der Erfindung sind daher Zusammensetzungen enthaltend (a) ein gegen oxidativen,

thermischen oder/und aktinischen Abbau empfindliches organisches Material und (b) mindestens eine Verbindung der Formeln I und/oder II, sowie die Verwendung von Verbindungen der Formeln I und II zum Stabilisieren von organischem Material gegen oxidativen, thermischen oder aktinischen Abbau.
Die Erfindung umfaßt ebenfalls ein Verfahren zum Stabilisieren von organischem Material gegen thermischen, oxidativen oder/und aktinischen Abbau, dadurch gekennzeichnet, daß man diesem Material mindestens eine Verbindung der Formeln I und/oder II zusetzt.

Von besonderem Interesse ist die Verwendung von Verbindungen der Formeln I und II als Stabilisatoren in synthetischen organischen Polymeren.

Vorzugsweise handelt es sich bei den zu schützenden organischen Materialien um natürliche, halbsynthetische oder bevorzugt synthetische organische Materialien. Besonders bevorzugt sind synthetische organische Polymere oder Gemische solcher Polymere, insbesondere thermoplastische Polymere wie Polyolefine, vor allem Polyethylen und Polypropylen (PP). Ebenfalls besonders bevorzugte organische Materialien sind Überzugszusammensetzungen. Unter photographischen Materialien sind insbesondere die Materialien zu verstehen, die in Research Disclosure 1990, 31429 (Seiten 474-480) für die photographische Reproduktion und andere Reproduktionstechniken beschrieben sind. Im Sinne der Erfindung vorteilhaft zu stabilisierende Überzugszusammensetzungen sind beispielsweise beschrieben in Ullmann's Encyclopedia of Industrial Chemistry, 5. Ed., Vol. A18, pp. 359-464, VCH Verlagsgesellschaft, Weinheim 1991; solche Überzugszusammensetzungen enthalten häufig weitere Additive, beispielsweise solche, wie weiter unten beschrieben, insbesondere UV-Absorber.

Allgemein werden die Verbindungen der Formel I und/oder II dem zu stabilisierenden Material in Mengen von 0,01 bis 10 %, bevorzugt 0,01 bis 5 %, insbesondere 0,01 bis 2 %, bezogen auf das Gesamtgewicht der stabilisierten Zusammensetzung, zugesetzt. Besonders bevorzugt ist der Einsatz der erfindungsgemäßen Verbindungen in Mengen von 0,05 bis 1,5 %, vor allem 0,1 bis 1,5 %.

Die Einarbeitung in die Materialien kann beispielsweise durch Einmischen oder Aufbringen der Verbindungen der Formel I und/oder II und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten Verbindung auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der Verbindungen der Formel I und/oder II in Polymere besteht in deren Zugabe vor, während oder unmittelbar nach der Polymerisation der entsprechenden Monomeren bzw. vor der Vernetzung. Die Verbindung der Formel I kann dabei als solche, aber auch in encapsulierter Form (z.B in Wachsen, Ölen oder Polymeren) zugesetzt werden. Im Falle der Zugabe vor oder während der Polymerisation können die Verbindungen der Formel I und/oder II auch als Regulator für die Kettenlänge der Polymere (Kettenabbrecher) wirken.

Die Verbindungen der Formel I und/oder II können auch in Form eines Masterbatches, der diese Verbindung beispielsweise in einer Konzentration von 2,5 bis 25 Gew.-% enthält, den zu stabilisierenden Kunststoffen zugesetzt werden.

Zweckmäßig kann die Einarbeitung der Verbindungen der Formel I und/oder II nach folgenden Methoden erfolgen:

- als Emulsion oder Dispersion (z.B. zu Latices oder Emulsionspolymeren),
- als Trockenmischung während des Vermischens von Zusatzkomponenten oder Polymermischungen,
- durch direktes Zugeben in die Verarbeitungsapparatur (z.B. Extruder, Innenmischer usw.),
- als Lösung oder Schmelze.

Erfindungsgemäße Polymerzusammensetzungen können in verschiedener Form angewendet bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Neben den Verbindungen der Formel I und/oder II können die erfindungsgemäßen Zusammensetzungen zusätzlich herkömmliche Additive enthalten, wie beispielsweise die unten angegebenen.

Die herkömmlichen Additive werden zweckmäßig in Mengen von 0,1-10, beispielsweise 0,2-5 Gew.-%, bezogen auf das zu stabilisierende Polymer, eingesetzt.

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-($\alpha$-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1-methyl-undec-1-yl)-phenol, 2,4-Dimethyl-6-(1-methyl-heptadec-1-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecyl-thiomethyl-4-nonylphenol.

1.3. Hydrochinone und alkylierte Hydrochinone, z.B. 2,6-Di-tert-butyl-4-methoxyphenol, 2,5-Di-tert-butylhydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit,

Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis- (3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2'4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2, 2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis- salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis- (2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri- tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3 ,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die nachfolgenden Beispiele illustrieren die Erfindung weiter. Alle Angaben in Teilen oder Prozenten, in den Beispielen ebenso wie in der übrigen Beschreibung sowie in den Patentansprüchen, beziehen sich auf das Gewicht, sofern nichts anderes angegeben ist. In den Beispielen werden die folgenden Abkürzungen verwendet:
GC: Gaschromatographie;
HPLC Hochdruck-Flüssigchromatographie;
GPC: Gelpermeationschromatographie;
MALDI: Matrix Assisted Laser Desorption Ionization;
DSC: Differential-Thermoanalyse;
DMF: Dimethylformamid;
THF: Tetrahydrofuran;
DMSO: Dimethylsulfoxid;
Tg: Glastemperatur;
$M_n$: Zahlenmittel der Molmasse (Einheit g/Mol);
$M_w$: Massenmittel der Molmasse (Einheit g/Mol).

Herstellungsbeispiele (A, B)

A) Herstellung der Epoxide

A1) Herstellung von 1-Benzyl-4-(2,3-epoxypropoxy)-2,2,6,6-tetramethylpiperidin

In einem 1,5 l Sulfierkolben, ausgerüstet mit Glasrührer, Innenthermometer, Tropftrichter und Wasserkühler werden 200 g Natriumhydroxid in 200 ml Wasser gelöst. Man gibt dann 500 ml Toluol, 32,2 g (0,1 Mol) Tetrabutylammoniumbromid und 247,4 g (1 Mol) 1-Benzyl-4-hydroxy-2,2,6,6-tetramethylpiperidin dazu.

Bei 50°C-55°C tropft man 185 g (2 Mol) Epichlorhydrin dazu, wobei die Mischung stark gerührt wird und hält die Temperatur anschliessend unter Rühren für 2-3 Stunden bei 55°C. Man giesst die Mischung dann auf 2 1 Eiswasser und extrahiert zweimal mit je 50 ml Essigester. Die org. Phase wird getrocknet und eingedampft. Der Rückstand wird bei 120°C ($8 \cdot 10^{-3}$ Torr) destilliert.
Ausbeute: 165 g (54 %)
HPLC-Analyse: 96 % Reinheit

| Mikroanalyse | | |
|---|---|---|
| | berechnet | gefunden |
| C | 75.21 | 75.17 |
| H | 9.63 | 9.58 |
| N | 4.62 | 4.71 |

$^1$H-NMR ($CDCl_3$):
0,98 ppm und 1,11 ppm (12 H,s): $CH_3$-
1,44-1,54 ppm und 1,89-1,97 ppm (4 H, m): -$CH_2$- des Piperidinrings
2,62-2,64 ppm und 2,79-2,83 ppm (2 H, m): $CH_2$-Epoxidring
3,13-3,19 ppm (1 H, m): CH-Epoxidring
3,45-3,51 und 3,77-3,81 ppm (2 H, m): -O-$CH_2$-
3,68-3,80 ppm (1 H, m): -CH- des Piperidinrings
3,81 ppm (2 H, s): N-$CH_2$-
7,11-7,43 ppm (5 H, m): aromat. Protonen

A2) Herstellung von 1,2,2,6,6-Pentamethyl-4-(2,3-epoxypropoxy)-piperidin

In einem 2,5 l Sulfierkolben mit mechanischem Rührer, Kühler und 500 ml Tropftrichter werden unter Argonatmosphäre 300 g Natriumhydroxid (7,5 Mol) in 300 ml Wasser gelöst. Dazu gibt man 750 ml Toluol, 48,4 g (0,15 Mol) Tetrabutylammoniumbromid und 257 g (1,5 Mol) 4-Hydroxy-1,2,2,6,6-pentamethylpiperidin.

Bei 60°C werden 347 g Epichlorhydrin (3,75 Mol) innerhalb 1,5 Stunden zugetropft, anschließend wird die Temperatur für weitere 4 Stunden unter Rühren bei 60°C gehalten.

Die Reaktionslösung wird auf 3 l Eiswasser gegossen, die organische Phase abgetrennt, mit Natriumsulfat getrocknet und eingedampft.

Bei 0,05 Torr wird über eine Vigreuxkolonne destilliert und die Fraktion mit Siedepunkt 71-72°C gesammelt.
Ausbeute: 205 g (60 %) GC: > 99 %

| Mikroanalyse | | |
|---|---|---|
| | berechnet | gefunden |
| C | 68.68 | 68.64 |
| H | 11.07 | 11.21 |
| N | 6.16 | 6.32 |
| Cl | 0.0 | 0.0 |

$^1$H-NMR ($CDCl_3$):
1,02 und 1,16 ppm (12 H, s): $CH_3$- Gruppen des Piperidinrings
1,32-1,4 ppm und 1,83-1,91 ppm (4 H, m): $-CH_2-$ Gruppen des Piperidinrings
2,23 ppm (3 H, s): N-$CH_3$
2,60-2,62 ppm und 2,78-2,82 ppm (2 H, m): $CH_2$-Gruppe des Epoxidrings
3,11-3,16 ppm (1 H, m): CH des Epoxidrings
3,42-3,47 und 3,71-3,76 ppm (2 H, m): -O-$CH_2$-Gruppe
3,57-3,67 ppm (1 H, m): -CH-O des Piperidinrings

A3) Herstellung von 1-Octyloxy-4-(2,3-epoxypropoxy)-2,2,6,6-tetramethylpiperidin

In einem 750 ml Sulfierkolben mit Glasrührer, Innenthermometer, Tropftrichter und Kühler werden 100 g Natriumhydroxid in 100 ml Wasser gelöst. Dazu gibt man 71,4 g (0,25 Mol) 1-Octyloxy-4-hydroxy-2,2,6,6-tetramethylpiperidin und 16,1 g (50 mmol) Tetrabutylammoniumbromid.
Bei 30°C wird die Mischung stark gerührt. Nun tropft man eine Lösung aus 71,4 g (0,25 Mol) 1-Octyloxy-4-hydroxy-2,2,6,6-tetramethylpiperidin in 277,5 g (3 Mol) Epichlorhydrin bei 33-35°C dazu.
Unter starkem Rühren lässt man bei 35°C 24 Stunden rühren. Man giesst auf 1 l Eiswasser, gesättigt mit Natriumchlorid, extrahiert die organische Phase mit 500 ml Essigester und trocknet über Natriumsulfat.
Nachdem das Lösungsmittel vom Rotovap abgedampft wurde, wird der Rückstand bei 130-134°C (0.01 Torr) destilliert.
Ausbeute: 109.8 g (64 %)

| Mikroanalyse | | |
|---|---|---|
| | berechnet | gefunden |
| C | 71.53 | 71.42 |
| H | 12.36 | 12.46 |
| N | 4.91 | 4.91 |

$^1$H-NMR ($CDCl_3$):
0,83-0,93 ppm (3 H, m): $CH_3$ (Octyl)
1,11-1,17 ppm (12 H, m): $CH_3$ (Piperidin)
1,28-1,81 ppm (18 H, m): $CH_2$ (Octyl, Piperidin)
2,59-2,61 und 2,77-2,81 ppm (2 H, m): $CH_2$ (Epoxidring)
3,10-3,14 ppm (1 H, m): CH (Epoxidring)
3,32-3,44 und 3,68-3,72 ppm (2 H, m): $CH_2$ (Epoxid)
3,56-3,68 ppm (1 H, m): CH (Piperidin).

A4) Herstellung von 2,2,6,6-Tetramethyl-4-(2,3-epoxypropoxy)-piperidin

In einem 750 ml Sulfierkolben mit mechanischem Rührer, Kühler, Thermometer und 100 ml Tropftrichter werden unter Argon 64,0 g Natriumhydroxid (1,6 Mol) in 64 ml Wasser gelöst. Dazu gibt man 170 ml Toluol, 10,3 g (31,8 mmol) Tetrabutylammoniumbromid und 50 g (318 mmol) 4-Hydroxy-2,2,6,6-tetramethylpiperidin.

Bei 45°C werden 58,8 g (636 mmol) Epichlorhydrin zugetropft. Man lässt dann bei 50°C während 4 Stunden rühren. Das Reaktionsgemisch wird auf Raumtemperatur gekühlt und auf 1 l Eiswasser gegossen, die organische Phase abgetrennt, mit Natriumsulfat getrocknet und am Rotationsverdampfer eingeengt. Der Rückstand wird bei $8.10^{-3}$ Torr destilliert; Siedepunkt: 48°C. Man erhält 28 g (41 % d. Th.) des Titelprodukt in einer Reinheit von 98 % (GC).

| Mikroanalyse: | | |
|---|---|---|
| | berechnet | gefunden |
| C | 67.57 | 67.73 |
| H | 10.87 | 10.92 |
| N | 6.57 | 6.51 |

H-NMR($CDCl_3$):
0,577 ppm (1 H): NH
0,81-0,98 ppm (2 H, m): $CH_2$ (Piperidinring)
1,01 und 1,05 ppm (12 H, s): $CH_3$-Gruppen
1,77-1,87 ppm (2 H, m): $CH_2$ (Piperidinring)
2,47-2,50 und 2,66-2,69 ppm (2 H, m): $CH_2$ (Epoxidring)
2,99-3,04 ppm (1 H, m): CH (Epoxidring)
3,31-3,37 und 3,61-3,67 ppm (3 H, m): $CH_2$ (Epoxid) und CH-O (Piperidinring)

A5) 1-Cyclohexyloxy-2,2,6,6-tetramethyl-4-(2,3-epoxypropoxy)piperidin

A5a) 4-Acetyloxy-2,2,6,6-tetramethylpiperidin

In einem 10 l Planschliffgefäss mit mechanischem Rührer, Thermometer, Kühler und Tropftrichter werden unter Stickstoff 786,5 g (5 Mol) 4-Hydroxy-2,2,6,6-tetramethylpiperidin vorgelegt. Dazu gibt man 300 g (5 Mol) Essigsäure und 1531 g (15 Mol) Essigsäureanhydrid. Man tropft langsam etwa 10 Tropfen konzentrierter Schwefelsäure dazu und lässt 12 Stunden bei 60°C rühren.
Bei einer Innentemperatur von weniger als 30°C gibt man eine Lösung von 1,2 kg Natriumoxid in 3 1 Wasser dazu.
Die organische Phase wird zweimal mit 1 l Diethylether extrahiert, über Natriumsulfat getrocknet und das Lösungsmittel abgedampft.
Am Wasserstrahlvakuum wird die Substanz destilliert: Siedepunkt 103°C/15 Torr.
Ausbeute: 700 g (70 %)
GC-Reinheit > 95 %

| Mikroanalyse | | |
|---|---|---|
| | berechnet | gefunden |
| C | 66.29 | 66.03 |
| H | 10.62 | 10.74 |
| N | 7.03 | 6.93 |

IR (KBr-Platten): C=O bei 1740 $cm^{-1}$
$^1$H-NMR ($CDCl_3$):
1,03-1,18 ppm (2 H,m): -$CH_2$-
1,15 ppm (6 H, s): $CH_3$-C

1,24 ppm (6 H, s): $CH_3$-C
1,89-1,95 ppm (2 H, m): -$CH_2$-
2,03 ppm (3 H, s): $CH_3COO$-

A5b) 1-Cyclohexyloxy-2,2,6,6-tetramethyl-4-acetoxypiperidin

In einem 1,5 l Sulfierkolben mit Magnetrührer, Wasserabscheider, Thermometer und Tropftrichter wird unter Stickstoff 60 g (301 Mol) 4-Acetoxy-2,2,6,6-tetramethylpiperidin in 300 ml Cyclohexan gelöst. Dazu gibt man 4,3 g (30 mmol) Molybdänoxid. 154 g (1,2 Mol) einer wässrigen 70 %igen Lösung von t-Butylhydroperoxid werden dreimal mit je 35 ml Cyclohexan extrahiert, die organische Phase über Natriumsulfat getrocknet und in den Tropftrichter transferiert.
Man erwärmt die Reaktionsmischung zum Rückfluss und tropft inerhalb von 2 Stunden die t-Butylhydroperoxidlösung dazu. Nach weiteren 2 Stunden ist die Wasserabspaltung abgeschlossen. Die Mischung wird über Nacht auf Rückflusstemperatur gehalten. Dann wird auf 25°C abgekühlt und vom Katalysator abfiltriert. Man versetzt mit Eis, gibt zur Vernichtung von überschüssigem Hydroperoxid etwas Natriumsulfit dazu und trennt dann die organische Phase ab. Diese wird mit Wasser gewaschen, über Natriumsulfat getrocknet und das Lösungsmittel am Rotavap entfernt.
Ausbeute: 85 g (95 %)
Die Flüssigkeit wird zu Analysezwecken im Kugelrohr destilliert. Die farblose Flüssigkeit siedet bei 115°C/0,05 Torr.
GC-Analyse: 96 %

| Mikroanalyse | | |
|---|---|---|
| | berechnet | gefunden |
| C | 68.65 | 68.57 |
| H | 10.51 | 10.49 |
| N | 4.71 | 4.56 |

$^1$H-NMR ($CDCl_3$):
1,19 ppm (12 H,s): $CH_2$-C
1,08-2,02 ppm (14 H, m): -$CH_2$-
2,01 ppm (3 H, s): $CH_3$-C00
4,95-5,05 ppm (1 H, m):

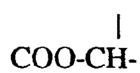

A5c) 1-Cyclohexyloxy-2,2,6,6-tetramethyl-4-hydroxypiperidin

In einem Erlenmeyerkolben werden 24 g (428 mmol) Kaliumhydroxid in 600 ml Methanol gelöst. Zur warmen Lösung werden 85 g (286 mmol) 1-Cyclohexyloxy-2,2,6,6-tetramethyl-4-acetoxypiperidin unter Rühren zugegossen. Anschliessend giesst man auf Eis und extrahiert mit Diethylether. Nach dem Trocknen über Natriumsulfat wird Ether abgedampft und der dickflüssige Rückstand in 300 ml Acetonitril warm gelöst. Man filtriert und lässt kristallisieren; Ausbeute: 50 g (68 %).
Die farblose Substanz hat einen Schmelzpunkt von 78,5°C; Reinheit gemäß GC-Analyse: 96 %.

| Mikroanalyse | | |
|---|---|---|
| | berechnet | gefunden |
| C | 70.54 | 70.55 |
| H | 11.45 | 11.59 |
| N | 5.48 | 5.36 |

$^1$H-NMR ($CDCl_3$):
1,14 ppm und 1,24 ppm (12 H,s): $CH_3$
1,08-2,07 ppm (14 H, m): $-CH_2-$
3,57 - 3,62 ppm (1 H, m):
4,95-5,05 ppm (1 H, m):

|
N-O-CH-

3,90 - 3,99 ppm (1 H, m):

|
-O-CH-

A5d) 1-Cyclohexyloxy-2,2,6,6-tetramethyl-4-(2,3-epoxypropoxy)piperidin

In einem 750 ml Sulfierkolben, ausgerüstet mit Rührer, Thermometer und Tropftrichter wird unter Argon 78,4 g (1,96 Mol) Natriumhydroxid in 80 ml Wasser gelöst. Dazu gibt man 100 g (392 mmol) 1-Cyclohexyloxy-2,2,6,6-tetramethyl-4-hydroxypiperidin und 12,6 g (39,2 mmol) Tetrabutylammoniumbromid gelöst in 250 ml Toluol. Es wird auf 50-55°C erwärmt und während 45 Minuten werden tropfenweise 90,7 g (980 mmol) Epichlorhydrin zugegeben. Dabei wird das Gemisch stark gerührt.
Man lässt bei 55°C weitere 3 Stunden rühren. Dann giesst man die Reaktionsmischung auf 1 Liter Eiswasser, trennt die organische Phase ab und wäscht diese einmal mit Wasser. Die organische Phase wird über Natriumsulfat getrocknet, mit Aktivkohle entfärbt, filtriert und eingedampft.
Der Rückstand wird über eine Vigreuxkolonne destilliert: Siedepunkt
116-117°C/0.06 Torr.
Ausbeute: 78,7 g (64 %)
GC-Reinheit: 98 %

| Mikroanalyse | | |
|---|---|---|
| | berechnet | gefunden |
| C | 69.41 | 69.34 |
| H | 10.68 | 11.24 |
| N | 4.50 | 4.39 |

$^1$H-NMR ($CDCl_3$):
1,13 ppm und 1,119 ppm (12 H, s) $CH_3-$
1,08 ppm - 2,04 ppm (14 H,m): $-CH_2-$
2,59 ppm - 2,81 ppm (2 H, m): $CH_2$-Epoxidring
3,10 ppm - 3,43 ppm (2 H, m): $CH_2$-Epoxid
3,56 ppm - 3,73 ppm (3 H, m): CH-Epoxidring und CH-O der Sechserringe

Weitere Epoxide werden in Analogie zu den oben beschriebenen Produkten A1 bis A5 hergestellt.

B) Herstellung der Addukte

B1) Addition von Bisphenol A an Epoxid A1

In einem 500 ml Rundkolben mit Magnetrührer, Kühler und Argonballon werden 50 g (165 mmol) des Epoxids A1, 17 g (75 mmol) Bisphenol A, 200 ml Xylol und 1,4 g (3,8 mmol) Ethyltriphenylphosphoniumbromid vorgelegt.

Die Mischung wird unter Rühren zum Rückfluss erwärmt und der Umsatz mit HPLC bestimmt.

Nach 15 Stunden giesst man auf Eis und extrahiert mit Toluol. Die organische Phase wird zweimal mit Wasser gewaschen und über Natriumsulfat getrocknet. Nach dem Abdampfen wird das Produkt über Kieselgel 60 (Hersteller: E. Merck, Darmstadt) mit Hexan/Essigester 2:1 chromatographiert und dann am Hochvakuum getrocknet. Man erhält das Produkt der Formel

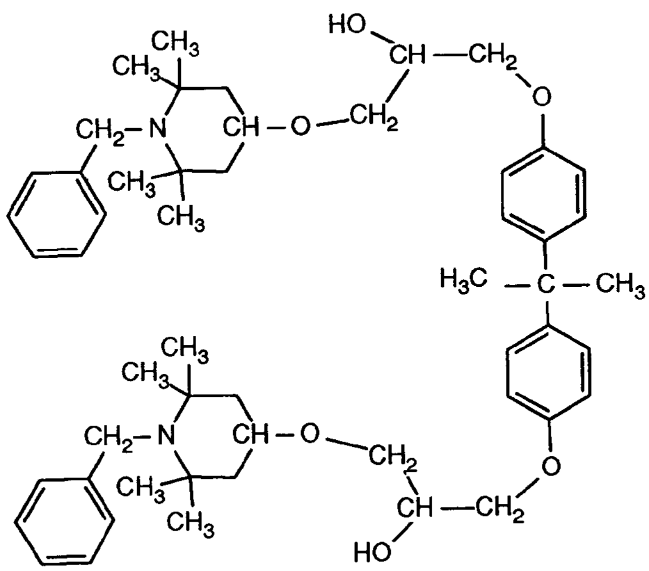

als farblose, hochviskose Flüssigkeit.
Ausbeute: 37 g (59 %)

| Mikroanalyse | | |
|---|---|---|
| | berechnet | gefunden |
| C | 76.22 | 76.21 |
| H | 8.93 | 8.96 |
| N | 3.35 | 3.12 |

$^1$H-NMR ($CDCl_3$):
0,97 und 1,09 ppm (24 H, s): $CH_3$ (Piperidin)
1,44-1,52 ppm und 1,89-1,95 ppm (8 H, m): $CH_2$ (Piperidin)
1,68 ppm (6 H, s): $CH_3$ (Bisphenol A)
2,63-2,65 ppm (2 H, d): OH
3,60-3,75 ppm (6 H, m): CH-O (Piperidin) und $CH_2$-O-Piperidin
3,80 ppm (4 H, s): N-$CH_2$
4,01-4,08 (4 H, d) ar-O-$CH_2$
4,10-4,16 (2 H, m):

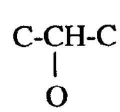

6,81-6,84 und 7,11-7,14 ppm (8 H, m): H-ar (Bisphenol A)
7,13-7,42 ppm (10 H, m): H-ar (Benzylgruppe)

Im Bereich zwischen 2,7 und 3,5 ppm sind keine Signale sichtbar, d.h. es wurde eine vollständige Umsetzung der Epoxidgruppen erreicht.

B2) Addition von Resorcin an das Epoxid A2

In einem 1 l Rundkolben mit Magnetrührer, Kühler und Argonballon werden 100 g (440 mmol) des Epoxids A2, 22 g (200 mmol) Resorcin, 3,7 g (10 mmol) Ethyltriphenylphosphoniumbromid und 400 ml Xylol vorgelegt. Man erhitzt unter Rühren für 24 Stunden auf Rückfluss. Die erkaltete, klare Lösung wird mit 1 N Natronlauge und mit Wasser gewaschen und über Natriumsulfat getrocknet. Nach Behandeln mit Aktivkohle wird die Lösung eingedampft und am Hochvakuum getrocknet.

Man erhält in quantitativer Ausbeute das Produkt der Formel

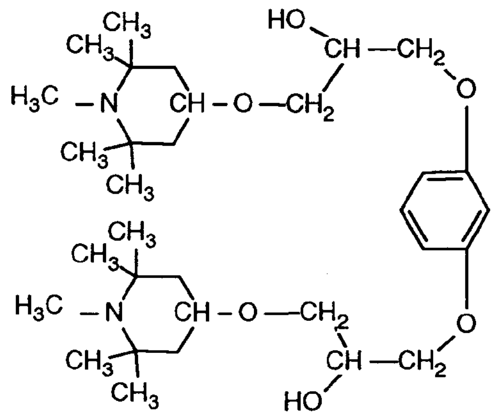

als hochviskose klare Flüssigkeit.

| Mikroanalyse | | |
|---|---|---|
| | berechnet | gefunden |
| C | 68.05 | 67.49 |
| H | 9.99 | 10.04 |
| N | 4.96 | 4.46 |

$^1$H-NMR ($CDCl_3$): zeigt keine Epoxidgruppen mehr
GPC (THF): $M_n$: 522
$M_w$: 579

B3) 4-(3-Acryloxy-2-hydroxypropyloxy)-1,2,2,6,6-pentamethylpiperidin

In einem 2,5 l Sulfierkolben mit Magnetrührer, Thermometer und Kühler werden unter Argon 375 g (1,65 Mol) 4-(2,3-Epoxypropoxy)-1,2,2,6,6-pentamethylpiperidin in 1,5 l Xylol vorgelegt. Dazu gibt man 108 g (1,5 Mol) Acrylsäure und 27,8 g (75 mmol) Ethyltriphenylphosphoniumbromid.

Bei 70°C lässt man während 18 Stunden reagieren. Das abgekühlte Produkt giesst man auf Eiswasser und trennt die org. Phase ab. Die organische Phase wird zweimal mit 1 n Natronlauge gewaschen, getrocknet und eingedampft.

Der Rückstand wird über eine kurze Vigreuxkolonne destilliert. Man erhält 193 g (43 %) einer klaren Flüssigkeit vom Siedepunkt 119-125°C (=0,06 Torr).

| Mikroanalyse | | |
|---|---|---|
| | berechnet | gefunden |
| C | 64.18 | 63.50 |
| H | 9.76 | 9.76 |
| N | 4.68 | 4.67 |

$^1$H-NMR ($CDCl_3$):
1,02 und 1,16 ppm (12 H,s): $CH_3$ (Piperidin)
1,33-1,40 und 1,84-1,89 ppm (4 H, m): $CH_2$ (Piperidin)
2,23 ppm (3 H, s): N-$CH_3$
2,59 ppm (1 H, s): OH
3,46-3,63 und 3,71-3,72 und 3,84-3,86 und 3,98-4,05 und 4,18-4,29 ppm (6 H, m):

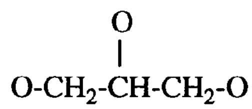

und CH (Piperidin)
5,84-5,88 und 6,12-6,22 und 6,42-6,48 ppm (3 H, m): CO-CH=$CH_2$

B4) 4-(3-Methacryloxy-2-hydroxypropyloxy)-1,2,2,6,6-pentamethylpiperidin

In einem 1,5 l Sulfierkolben mit Magnetrührer, Thermometer und Kühler werden unter Schutzgas 250 g (1,1 Mol) 4-(2,3-Epoxypropoxy)-1,2,2,6,6-pentamethylpiperidin in 1 l Xylol gelöst. Dazu gibt man 86 g (1 Mol) Methacrylsäure und 18,6 g (50 mmol) Ethyltriphenylphosphoniumbromid.

Die Mischung wird bei 70°C während 18 Stunden gerührt. Nach dem Abkühlen giesst man auf Eiswasser und trennt die organische Phase ab. Diese wird zweimal mit 1 n Natronlauge gewaschen und getrocknet. Das Lösungsmittel wird am Rotationsverdampfer abgezogen und der Rückstand bei 0,07 Torr destilliert.

Man erhält 176,5 g (56 %) einer klaren Flüssigkeit, welche bei 128-130°C/0,07 Torr siedet.

| Mikroanalyse | | |
|---|---|---|
| | berechnet | gefunden |
| C | 64.14 | 64.72 |
| H | 9.97 | 10.00 |
| N | 4.47 | 4.33 |

$^1$H-NMR ($CDCl_3$):
1,01 und 1,16 ppm (12 H,s): $CH_3$ (Piperidin)
1,32-1,40 und 1,83-1,88 ppm (4 H, m): $CH_2$ (Piperidin)
1,96 ppm (3 H, s): $CH_3$ (Methacrylat)
2,23 ppm (3 H, s): $CH_3$-N
2,68 ppm (1 H, s): OH
3,46-4,26 ppm (6 H, m):

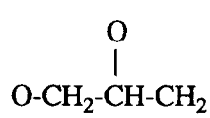

und CH (Piperidin)

5,59 und 6,14 ppm (2 H, s): $CH_2$=C

B5) 4-(3-[4-Vinylbenzoyloxy]-2-hydroxypropyloxy)-1,2,2,6,6-pentamethylpiperidin

In einem 500 ml Rundkolben, ausgerüstet mit Magnetrührer, Kühler, Thermometer und Argonballon werden 32,6 g (220 mmol) 4-Vinylbenzoesäure, 45,5 g (200 mmol) 4-(2,3-Epoxypropoxy)-1,2,2,6,6-pentamethyl piperidin, 3,7 g (10 mmol) Ethyltriphenylphosphoniumbromid und 200 ml Xylol vorgelegt.

Unter Schutzgas wird die Mischung auf 65°C erwärmt. Die klare Lösung wird während 15 Stunden bei 65°C gehalten und dann auf Eis gegossen. Die organische Phase wird bei 0°C zweimal mit 1 n Natronlauge gewaschen, über Natriumsulfat getrocknet und eingedampft.

Der Rückstand wird über eine Kieselgelsäule (THF) gereinigt und am Hochvakuum bei 60°C getrocknet.

Man erhält 52,7 g (70 %) einer klaren, viskosen Flüssigkeit.

| Mikroanalyse | | |
|---|---|---|
| | berechnet | gefunden |
| C | 70.37 | 69.89 |
| H | 8.86 | 9.22 |
| N | 3.73 | 3.79 |

1H-NMR ($CDCl_3$):
1,01 und 1,15 ppm (12 H,s): $CH_3$
1,33-1,43 und 1,84-1,89 ppm (4 H, m): $CH_2$ (Piperidin)
2,23 ppm (3 H, s): N-$CH_3$
2,81 ppm (1 H, s): OH
3,42-4,40 ppm (6 H, m):

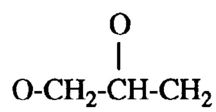

und CH (Piperidin)
5,36-5,40 ppm und 5,83-5,89 ppm (2 H, d): $CH_2$=
6,70-6,79 ppm (1 H, q): CH=
7,44-7,47 ppm und 7,99-8,02 ppm (4 H, d): Aromat

B6) 4-(3-[4-Vinylphenoxy]-2-hydroxypropyloxy)-1,2,2,6,6-pentamethylpiperidin

Analog Beispiel B2) werden 28,8 g (240 mMol) 4-Vinylphenol mit 45,5 g (200 mMol) 4-(2,3-Epoxypropoxy)-1,2,2,6,6-pentamethylpiperidin umgesetzt.
Ausbeute: 66 g (95 %)

| Mikroanalyse: | | % C | % H | % N |
|---|---|---|---|---|
| | berechnet | 72,58 | 9,57 | 4,03 |
| | gefunden | 72,67 | 9,53 | 3,89 |

$^1$H-NMR ($CDCl_3$):
1,01 und 1,15 ppm (12H, s): $CH_3$ (Piperidin)
1,32-1,41 ppm und 1,84-1,89 ppm (4H, m): $CH_2$ (Piperidin)
2,23 ppm (3H, s): N-$CH_3$
2,72 ppm (1H, s): OH
3,54-3,69 ppm (3H, m): Pip-O-$CH_2$-CH-
3,98-4,03 ppm (2H, d): ar-O-$CH_2$-

4,06-4,12 ppm (1H, m): >CH-O-
5,11-5,14 ppm und 5,58-5,64 ppm (2H, d): $CH_2$=
6,60-6,70 ppm (1H, q): CH=
6,86-6,89 ppm und 7,32-7,35 ppm (4H, d): H-Aromat

Beispiele B7-B12:

Entsprechend der unter B3 beschriebenen Methode werden die Addukte gemäß folgender Tabelle durch Umsetzung des entsprechenden Epoxids mit der entsprechenden Säure hergestellt:

| Beispiel | Addukt | Epoxid | Säure |
|---|---|---|---|
| B7 | 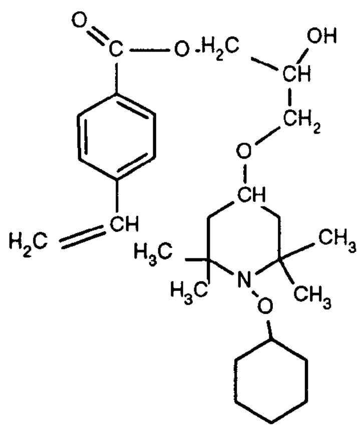 | 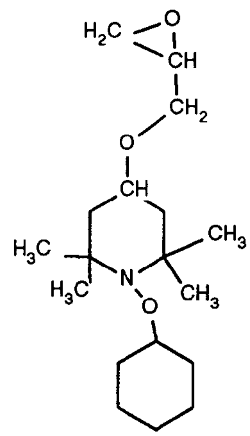 | 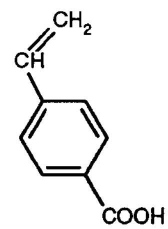 |
| B8 | 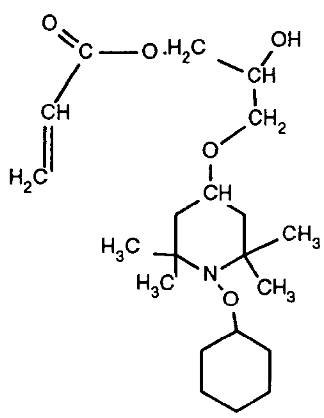 | 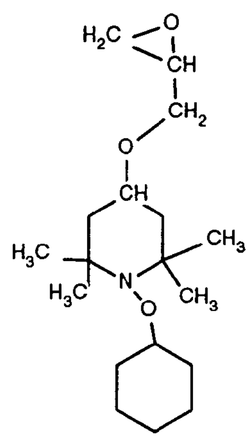 | 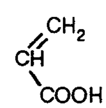 |

| Beispiel | Addukt | Epoxid | Säure |
|---|---|---|---|
| B9 | 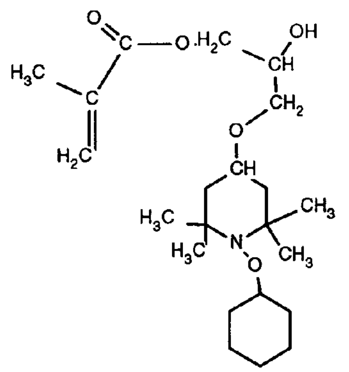 | 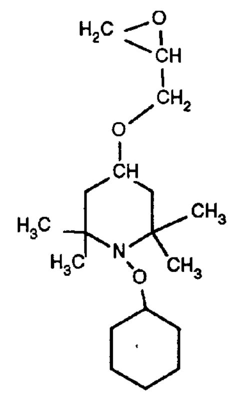 | 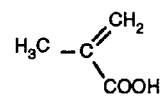 |
| B10 | 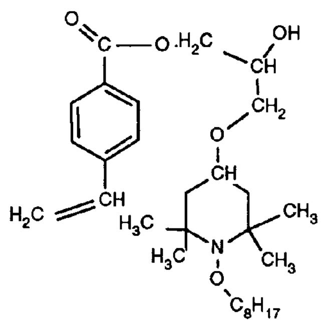 | 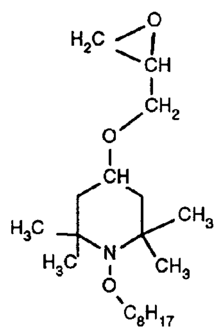 | 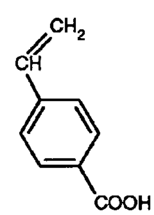 |
| B11 | 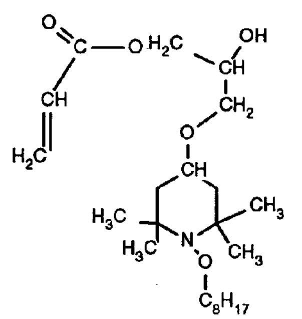 | 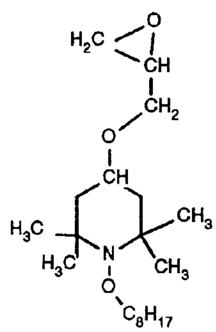 | 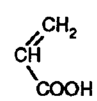 |
| B12 | 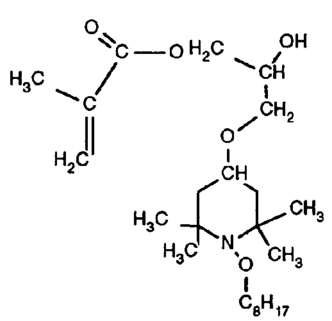 | 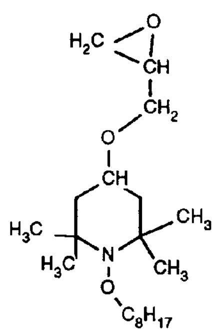 | 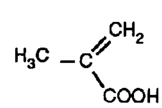 |

Beispiel B13: Bis[2-Hydroxypropyloxy-3(1,2,2,6,6-pentamethylpiperidin-4-yl)]-isophthalsäureester

In einem 1 l Rundkolben mit Magnetrührer und Rückflußkühler werden unter Argon 100 g (440 mmol) des Epoxids A2, 33 g (200 mmol) Isophthalsäure, 3,7 g (10 mmol) Ethyltriphenylphosphoniumbromid und 400 ml

Xylol vorgelegt.

Man erhitzt unter Rühren für 24 Stunden auf Rückfluss. Anschließend wird auf 0°C abgekühlt und die Lösung zweimal mit 1 n Natronlauge und einmal mit Wasser gewaschen und über Natriumsulfat getrocknet. Nach Behandeln mit Aktivkohle und Filtrieren wird die Lösung am Rotavap eingedampft und unter Hochvakuum bei 50°C getrocknet.

Man erhält das Titelprodukt in quantitativer Ausbeute; weitere Daten des Produktes finden sich in der folgenden Tabelle 1.

| Mikroanalyse | | |
|---|---|---|
| | berechnet | gefunden |
| C | 65.72 | 66.52 |
| H | 9.02 | 9.14 |
| N | 4.51 | 4.04 |

Beispiele B14-B25:

Entsprechend der in den Beispielen B2 und B13 beschriebenen Methode werden die weiteren in der Tabelle 1 aufgeführten Addukte B14 bis B25 hergestellt.
Spalte 2 gibt den Typ des verwendeten Ausgangsepoxids sowie dessen Molverhältnis zur verwendeten Carbonsäure bzw. Phenol im Endprodukt an; beispielsweise bedeutet 2xA2 in Zeile 1 der Tabelle 1 an, daß die Verbindung des Beispiels B 13 das Produkt aus 2 Mol Epoxid A2 pro Mol Isophthalsäure ist.

Die in der Tabelle verwendeten Abkürzungen bedeuten
GPC: Ergebnisse der Gelpermeationschromatographie
$M_n$: Zahlenmittel der Molmasse;
$M_w$: Massenmittel der Molmasse;
Tg: Glastemperatur.

Tabelle 1:

| Ausgangsstoffe und phys. Daten zu den Verbindungen der Beispiele 13-25 | | | | | | |
|---|---|---|---|---|---|---|
| Bsp Nr. | Epoxid | Carbonsäure/Phenol | Aubeute | GPC | | Produkt |
| | | | | $M_n$ | $M_w$ | |
| B13 | 2xA2 | Isophthalsäure | 100 % | 672 | 897 | viskose Flüssigkeit |
| B14 | 2xA2 | Bernsteinsäure | 23 % | 680 | 910 | viskose Flüssigkeit |
| B15 | 2xA2 | Bisphenol A | 100 % | 585 | 719 | viskose Flüssigkeit |
| B16 | 2xA2 | 1,4-Cyclohexandicarbonsäure | 100 % | 576 | 721 | Festkörper, wachsartig |
| B17 | 1xA2 | Stearinsäure | 82 % | 571 | 646 | viskose Flüssigkeit |
| B18 | 2xA2 | Sebacinsäure | 92 % | 692 | 861 | viskose Flüssigkeit |
| B19 | 1xA2 | Dodecansäure | 91 % | 657 | 841 | viskose Flüssigkeit |
| B20 | 1xA2 | Laurinsäure | 89 % | 398 | 442 | viskose Flüssigkeit |
| B21 | 2xA1 | Sebacinsäure | 96 % | 737 | 948 | viskose Flüssigkeit |
| B22 | 2xA3 | Sebacinsäure | 97 % | 850 | 1093 | viskose Flüssigkeit |
| B23 | 3xA2 | Trimesinsäure | 58 % | 760 | 980 | Festkörper Tg: 29°C |
| B24 | 3xA2 | Phloroglucin | 81 % | 900 | 1165 | Feskörper Tg: 31°C |
| B25 | 3xA2 | 1,3,5-Cyclohexantricarbonsäure | 62 % | 750 | 850 | Festkörper Tg: 20°C |

B26) Addukt aus 1,2,2,6,6-Pentamethyl-4-(2,3-epoxypropoxy)-piperidin und 1,2,4,5-Benzoltetracarbonsäure

10 g (39,3 mmol) 1,2,4,5-Benzoltetracarbonsäure, 35,8 g (157,4 mmol) 1,2,2,6,6-Pentamethyl-4(2,3-epoxypropoxy)-piperidin (= Epoxid A2) und 0,75 g (2 mmol) Ethyltriphenyl-phosphoniumbromid werden in 100 ml DMSO gelöst und unter Argon auf 150°C erwärmt. Nach 20 Stunden wird auf Raumtemperatur abgekühlt und auf Wasser gegossen. Es wird dreimal mit Essigester extrahiert und die organische Phase mit 1N Natronlauge gewaschen. Die organische Phase wird mit Aktivkohle und Natriumsulfat behandelt, filtriert und eingedampft. Der Rückstand wird in wenig Ether gelöst und in n-Hexan gefällt, Man erhält 5,5 g des Titelproduktes als schwach beiges Pulver;
Tg = 60-65°C.

| Mikroanalyse: | C | H | N |
|---|---|---|---|
| berechnet | 64,00 | 9,18 | 4,82 |
| gefunden | 63,97 | 9,28 | 4,77 |

Molekulargewicht (MALDI): $M_n$ = 1800 $M_w$ = 2100

B27) 4-(3-[4-Vinylahenoxy]-2-hydroxypropyloxy)-1-cyclohexyloxy-2,2,6,6-tetramethylpiperidin

Analog Beispiel B6) wird 4-Vinylphenol mit 4-(2,3-Epoxypropoxy)-1-cyclohexyloxy-2,2,6,6-tetramethylpiperidin (= Epoxid A5) unter Bildung des Titelproduktes umgesetzt.

B28) 4-(3-[4-Vinylphenoxy]-2-hydroxypropyloxy)-1-octyloxy-2,2,6,6-tetramethylpiperidin

Analog Beispiel B6) wird 4-Vinylphenol mit 4-(2,3-Epoxypropoxy)-1-octyloxy-2,2,6,6-tetramethylpiperidin (= Epoxid A3) unter Bildung des Titelproduktes umgesetzt.

C) Anwendungsbeispiele

Beispiel C1: Stabilisierung von Polypropylenplatten

Je 1 g des in Tabelle C1 angegebenen erfindungsgemäßen Stabilisators werden mit 1000 g Polypropylen vom Schmelzindex 4,0 g/10 Min. (230°C; 2.16 kg) des Herstellers Statoil, Stathelle, Norwegen, und den folgenden Zusätzen gemischt:

0,5 g Pentaerythrityl-tetrakis(3-[3',5'-di-tert.butyl-4'-hydroxyphenyl]-propionat);
1,0 g Tris(2,4-di-tert.butyl-phenyl)-phosphit;
1,0 g FILOFIN® Blue G (Pigment; Ciba Geigy S.p.A., Origgio, Italien);
1,0 g Calciumstearat.

Zu Vergleichszwecken wird eine Mischung ohne erfindungsgemäßen Stabilisator hergestellt.

Die Mischung wird bei 200-230°C extrudiert (Berstorff KE 30x32D). Das erhaltene Granulat wird im Spritzgußverfahren bei 200-220°C zu Platten von 2 mm Dicke verarbeitet.

Die Platten werden gemäß ASTM D 2565-85 in einem Weather-O-Meter® 65 WR (Atlas Electric Devices, Chicago, USA) bei einer Schwarztafeltemperatur von 63 ± 3 °C belichtet. Die Proben werden regelmäßig untersucht; der Beginn einer oberflächlichen Versprödung (Chalking) wird ermittelt. Die Belichtungszeit bis zum Auftreten der Versprödung ist der nachfolgenden Tabelle C1 zu entnehmen.

Tab. C1 :

| Belichtungsdauer bis Oberflächenversprödung | |
|---|---|
| Stabilisator aus Beispiel | Belichtungsdauer (h) |
| ohne | 620 |
| B2 | 2810 |
| B13 | 2810 |
| B14 | 2810 |
| B15 | 2420 |
| B16 | 2810 |

Die erfindungsgemäß stabilisierten Proben weisen eine hervorragende Lichtbeständigkeit auf.

Beispiel C2: Stabilisierung eines Zweischicht-Lackes

Ein Klarlack wird bereitet aus

| | |
|---|---|
| 54,5 | Teilen eines Acrylharzes (Uracron® 2263 XB, DSM Resin BV) (50 %ige Lösung in Xylol) |
| 16,3 | Teilen eines Melaminharzes (Cymel® 327, Amer. Cyanamid Co.) (90 %ige Lösung) |
| 19,4 | Teilen Xylol |
| 5,5 | Teilen Butylglykolacetat |
| 3,3 | Teilen Butanol und |
| 1,0 | Teil eines Verlaufshilfsmittels (Baysilon®A, Bayer AG) (1 %ige Lösung in Xylol). |

Die in den Tabelle C2 aufgeführten erfindungsgemäßen Stabilisatoren werden als Lösung in Xylol dem Klarlack zugegeben in einer Menge von 1,0 % bezogen auf den Feststoffgehalt des Klarlackes. Zu Vergleichszwecken wird eine Probe ohne Stabilisatoren hergestellt. Einem Teil der Proben wird neben dem erfindungsgemäßen Stabilisator noch ein UV-Absorber der Formel

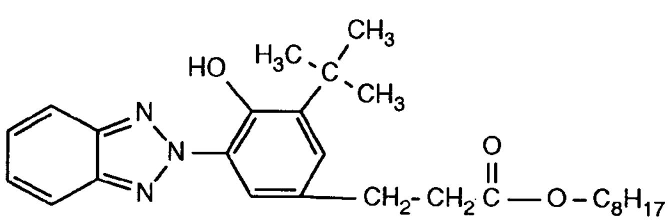

(= UVA) in einer Menge von 1,5 % bezogen auf den Feststoffgehalt des Klarlackes zugesetzt.

Die so bereiteten Lackproben werden mit einer Mischung aus Xylol, Butanol und Butylglykolacetat (13:6:1) auf Spritzfähigkeit verdünnt und auf ein vorbereitetes Aluminiumblech (coil coat, Automobilfüller, silbermetallic Basislack) aufgespritzt. Nach 15 Minuten Lagerung werden die Proben 30 Minuten bei 130°C gehärtet. Es resultiert eine Schichtdicke des Klarlackes von 40-50 μm.

Die Proben werden dann in einem UVCON-Bewitterungsgerät der Fa. Atlas (UVB-313 Lampen) bei einem Zyklus von 8 h UV-Bestrahlung bei 70°C und 4 h Kondensation bei 50°C bewittert. Nach jeweils 400 h Bewitterung wird der 20°-Glanz der Proben nach DIN 67530 gemessen. Die Ergebnisse sind in Tabelle C2 wiedergegeben.

Tab. C2: 20°-Glanz gemäß DIN 67530 nach der angegebenen Bewitterungsdauer

| Stabilisator | 0 h | 1200 h | 2400 h | 3600 h | 4800 h | 6000 h | 7200 h |
|---|---|---|---|---|---|---|---|
| keiner | 87 | 45 | 26 | * | * | * | * |
| aus B18 | 87 | 81 | 48 | 38 | 25 | * | * |
| aus B19 | 87 | 78 | 36 | 30 | 17 | * | * |
| aus B22 | 87 | 73 | 37 | 30 | 18 | * | * |
| aus B18 + UVA | 87 | 85 | 83 | 83 | 81 | 80 | 73 |
| aus B19 + UVA | 87 | 86 | 83 | 83 | 82 | 81 | 79 |
| aus B21 + UVA | 87 | 84 | 82 | 80 | 79 | 75 | 67 |
| aus B22 + UVA | 87 | 86 | 83 | 83 | 82 | 80 | 77 |

* Probe matt oder geschrumpft

Die Proben mit den erfindungsgemässen Stabilisatoren zeigen eine hervorragende Glanzhaltung.

Beispiel C3: Stabilisierung eines Zweischicht-Lackes

Die Lichtschutzmittel werden in 5-10 g Xylol eingearbeitet und in einem Klarlack folgender Zusammensetzung geprüft:

| | |
|---|---|
| Synthacryl® SC 303 [1)] | 27,51 |
| Synthacryl® SC 370 [2)] | 23,34 |
| Maprenal® MF 650 [3)] | 27,29 |
| Butylacetat/Butanol (37/8) | 4,33 |
| Isobutanol | 4,87 |
| Solvesso® 150 [4)] | 2,72 |
| Kristallöl K-30 [5)] | 8,74 |
| Verlaufshilfsmittel Baysilon® MA [6)] | 1,20 |
| | 100,00 g |

1) Acrylatharz, Fa. Hoechst AG; 65 % Lösung in Xylol/Butanol 26:9
2) Acrylatharz, Fa. Hoechst AG; 75 % Lösung in Solvesso®100[4)]
3) Melaminharz, Fa. Hoechst AG; 55 % Lösung in Isobutanol
4) Hersteller: Fa. ESSO
5) Hersteller: Fa. Shell
6) 1 % in Solvesso® 150; Hersteller: Fa. Bayer AG

Dem Klarlack werden 1 % Stabilisator zugesetzt, bezogen auf den Feststoffgehalt des Lackes. Als Vergleich dient ein Klarlack, der kein Lichtschutzmittel enthält.

Der Klarlack wird mit Solvesso® 100 auf Spritzfähigkeit verdünnt und auf ein vorbereitetes Aluminiumblech (coil coat, Füller, silbermetallic Basislack) gespritzt und bei 130°C 30 Minuten eingebrannt. Es resultiert eine Trockenfilmdicke von 40-30 μm Klarlack.

Die Proben werden dann in einem UVCON®-Bewitterungsgerät der Fa. Atlas Corp. (UVB-313 Lampen) bei einem Zyklus von 4 h UV-Bestrahlung bei 60°C und 4 h Kondensation bei 50°C bewittert.

Die Proben werden in regelmässigen Abständen auf Risse untersucht. Die Ergebnisse sind in Tabelle C3 aufgeführt.

Tab. C3:

| Bewitterungsdauer bis zur Rissbildung | |
|---|---|
| Stabilisator | Rissbildung nach |
| ohne | 1200 h |
| aus Beispiel B23 | >4800 h |

Die Probe mit dem erfindungsgemäßen Stabilisator weist eine hohe Beständigkeit gegenüber Rissbildung auf.

Beispiel C4: Stabilisierung eines photographischen Materials

0,087 g des Gelbkupplers der Formel

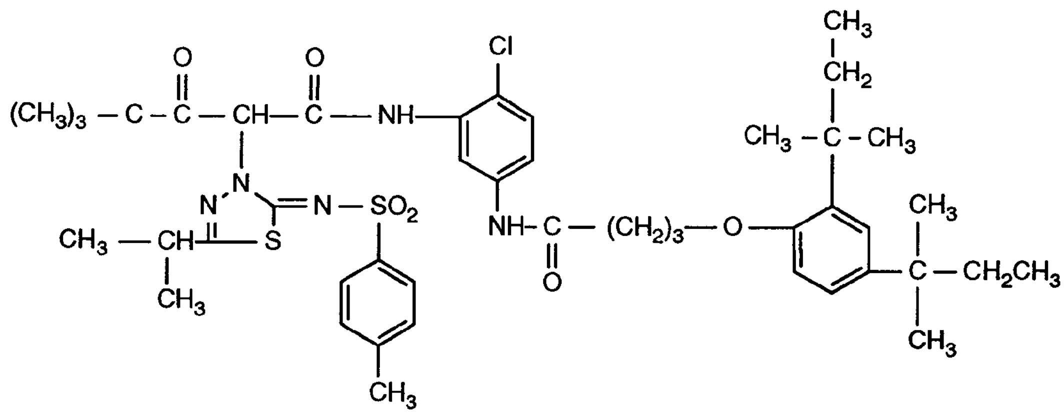

werden in 2,0 ml einer Lösung des erfindungsgemässen Stabilisators in Ethylacetat (2,25 g/100 ml) gelöst. Zu 1,0 ml dieser Lösung gibt man 9,0 ml einer 2,3%-igen wässrigen Gelatinelösung, die auf einem pH-Wert von 6,5 eingestellt ist, und 1,744 g/l des Netzmittels der Formel

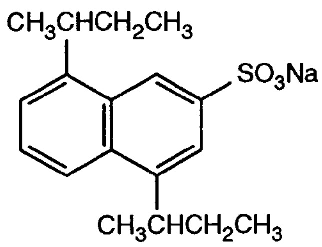

enthält.

Zu 5,0 ml der so erhaltenen Kuppleremulsion gibt man 2 ml einer Silberbromid-Emulsion mit einem Silbergehalt von 6,0 g/l sowie 1,0 ml einer 0,7%-igen wässrigen Lösung des Härters der Formel

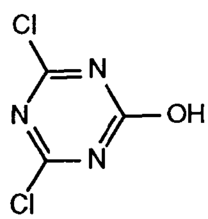

und vergiesst es auf ein 13 x 18 cm Kunststoff-beschichtetes Papier. Nach einer Härtungszeit von 7 Tagen werden die Proben hinter einem Silber-Stufenkeil mit 125 Lux•s belichtet und anschliessend im Ektaprint 2®-Prozess der Firma Kodak verarbeitet.

Die erhaltenen Gelbkeile werden in einem Atlas Weather-Ometer mit einer 2500 W-Xenonlampe hinter einem UV-Filter (Kodak 2C) mit total 60 k Joule/cm$^2$ bestrahlt.

Eine Probe ohne Stabilisator läuft als Standard mit.

Der bei der Bestrahlung eingetretene Farbdichteverlust beim Absorptionsmaximum des gelben Farbstoffes wird mit einem Densitometer TR 924A der Fa. Macbeth gemessen.

Der Lichtschutzeffekt ist aus dem Farbdichteverlust ersichtlich. Je kleiner der Dichteverlust, desto höher ist die Lichtschutzwirksamkeit.

Die erfindungsgemässen Stabilisatoren zeigen eine gute Lichtschutzwirkung.

**Patentansprüche**

1. Ester der Formel I

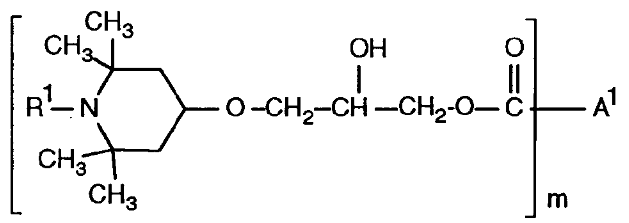

(I)

oder Phenolether der Formel II

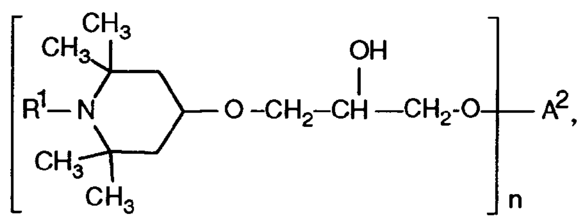

(II)

worin m und n jeweils eine ganze Zahl aus dem Bereich von 1 bis 6 darstellen;
$A^1$ einen m-wertigen Kohlenwasserstoffrest mit 1 bis 30 Kohlenstoffatomen darstellt; oder einen m-wertigen Kohlenwasserstoffrest mit 2 bis 30 Kohlenstoffatomen darstellt, der die Heteroatome Sauerstoff, Stickstoff und/oder Schwefel enthält und dessen freie Valenzen an Kohlenstoffatomen lokalisiert sind; wobei $A^1$ im Fall m = 2 zusätzlich eine direkte Bindung umfaßt;
$A^2$ einen n-wertigen aromatischen oder araliphatischen Kohlenwasserstoffrest mit 6 bis 30 Kohlenstoffatomen darstellt; oder einen n-wertigen aromatischen oder araliphatischen Kohlenwasserstoffrest mit 5 bis 30 Kohlenstoffatomen darstellt, der die Heteroatome Sauerstoff, Stickstoff und/oder Schwefel enthält und dessen n freie Valenzen an solchen Kohlenstoffatomen lokalisiert sind, die Bestandteil aromatischer Ringe sind;
$R^1$ Wasserstoff; einen Kohlenwasserstoff- oder Kohlenwasserstoffoxyrest mit 1 bis 36 Kohlenstoffatomen, der unsubstituiert oder durch $-CO-N(R^3)_2$ substituiert oder durch $-CO-N(R^3)-$ oder $-N(R^3)-CO-$ oder 1 bis 6 Sauerstoff- oder Schwefelatome unterbrochen ist; Benzoyl oder Naphtoyl, welches jeweils durch 1 bis 3 $C_1$-$C_4$-Alkyl- oder $C_1$-$C_4$-Alkoxyreste substituiert ist; oder $-CO-R^2$ bedeutet; wobei
$R^2$ $C_1$-$C_{18}$-Alkyl; $C_5$-$C_8$-Cycloalkyl; Phenyl; Naphthyl; $C_7$-$C_9$-Phenylalkyl; oder $C_{11}$-$C_{14}$-Naphthylalkyl bedeutet; und $R^3$ dieselben Bedeutungen umfaßt wie $R^2$ oder Wasserstoff bedeutet.

**2.** Verbindung der Formel I oder II gemäß Anspruch 1, worin
m und n 1, 2, 3 oder 4 sind;
$A^1$ im Fall m = 1 $C_1$-$C_{25}$-Alkyl darstellt, das unsubstituiert oder durch $C_5$-$C_8$-Cycloalkyl substituiert ist; oder $C_2$-$C_{25}$-Alkyl darstellt, das durch $C_5$-$C_8$-Cycloalkyl, -S-, -O- oder $-NR^2-$ oder verschiedener dieser Gruppen unterbrochen ist; oder $A^1$ im Fall m = 1 $C_5$-$C_8$-Cycloalkyl, das unsubstituiert oder mit $C_1$-$C_{12}$-Alkyl substituiert ist;
$C_6$-$C_8$-Cycloalkenyl, das unsubstituiert oder mit $C_1$-$C_{12}$-Alkyl substituiert ist; Phenyl, das unsubstituiert oder durch 1 bis 3 $C_1$-$C_4$-Alkyl-, $C_2$-$C_4$-Alkenyl- oder $C_1$-$C_4$-Alkoxyreste und/oder eine Gruppe der Formel

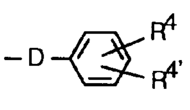

substituiert ist; oder $C_2$-$C_{25}$-Alkenyl; $C_3$-$C_{25}$-Alkenyl, das durch -O- unterbrochen ist; $C_7$-$C_{12}$-Phenylalkyl, das unsubstituiert oder durch $C_1$-$C_4$-Alkyl oder $C_2$-$C_4$-Alkenyl oder $C_1$-$C_4$-Alkoxy substituiert ist, bedeutet;
$A^1$ im Fall m = 2 eine direkte Bindung; $C_1$-$C_{18}$-Alkylen; $C_2$-$C_{18}$-Alkenylen;
$C_5$-$C_8$-Cycloalkylen, das unsubstituiert oder mit $C_1$-$C_{12}$-Alkyl substituiert ist;
$C_6$-$C_8$-Cycloalkenylen, das unsubstituiert oder mit $C_1$-$C_{12}$-Alkyl substituiert ist; Phenylen, das unsubstituiert oder durch 1 oder 2 $C_1$-$C_4$-Alkyl-, $C_2$-$C_4$-Alkenyl- oder $C_1$-$C_4$-Alkoxyreste und/oder eine Gruppe der Formel

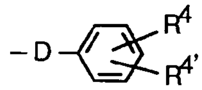

substituiert ist;
einen zweiwertigen Rest der Formel

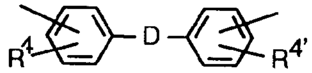

einen zweiwertigen heterozyklischen Rest aus der Gruppe Furan, Thiophen oder Pyrrol, welches am Stickstoffatom durch Wasserstoff oder den Substituenten $-R^2$ abgesättigt ist; oder $A^1$, im Fall m = 2, durch $C_5$-$C_8$-Cycloalkylen, Phenylen, -S-, -O- oder $-NR^2-$ oder verschiedener dieser Gruppen unterbrochenes $C_2$-$C_{18}$-Alkylen bedeutet; und
$A^1$ im Fall m = 3 $C_1$-$C_8$-Alkantriyl; $C_2$-$C_8$-Alkentriyl, Benzoltriyl; einen trivalenten Rest der Formel

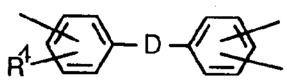 oder 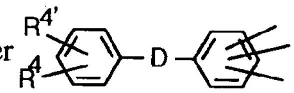 ;

eine trivalente Gruppe der Formel

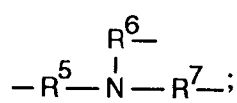

1,3,5-Triazin-2,4,6-triyl; $C_5$-$C_8$-Cycloalkantriyl; oder $C_2$-$C_{18}$-Alkantriyl, das durch -S-, -O- oder $-NR^2-$ unterbrochen ist und/oder durch tertiär -OH substituiert ist, bedeutet;
$A^1$ im Fall m = 4 einen Benzol-, Cyclopentyl- oder Cyclohexylrest mit 4 freien Valenzen oder einen tetravalenten Rest der Formel

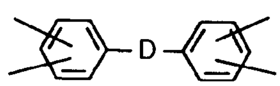 ,

einen tetravalenten Rest der Formel

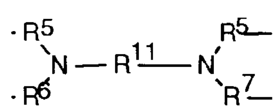 ,

oder $C_1$-$C_8$-Alkantetrayl bedeutet;
$A^2$ im Fall n = 1 Phenyl, das unsubstituiert oder durch 1 bis 3 $C_1$-$C_4$-Alkyl-, $C_2$-$C_4$-Alkenyl- oder $C_1$-$C_4$-Alkoxyreste und/oder eine Gruppe der Formel

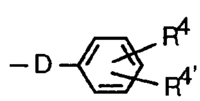

substituiert ist; einen Triazinylphenylrest der Formel

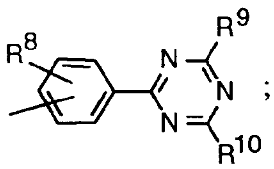

oder Naphthyl bedeutet;

$A^2$ im Fall n = 2 Phenylen, das unsubstituiert oder durch 1 oder 2 $C_1$-$C_4$-Alkyl-, $C_2$-$C_4$-Alkenyl- oder $C_1$-$C_4$-Alkoxyreste und/oder eine Gruppe der Formel

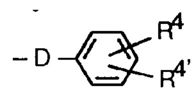

substituiert ist; einen zweiwertigen Rest der Formel

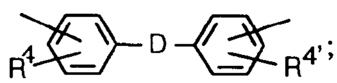

oder Naphthylen;

$A^2$ im Fall n = 3 Benzoltriyl; oder einen trivalenten Rest der Formel

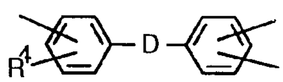
oder
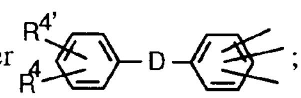

$A^2$ im Fall n = 4 einen Benzolrest mit 4 freien Valenzen oder einen tetravalenten Rest der Formel

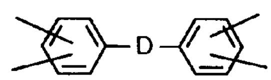

bedeutet;

oder $A^2$ einen n-wertigen Triphenyltriazinylrest der Formel

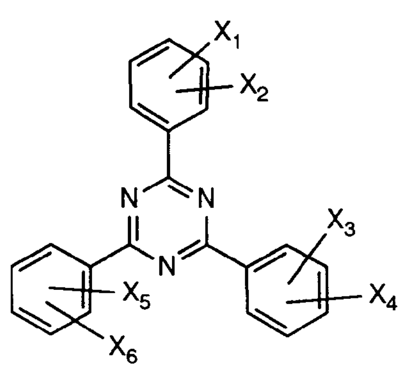

darstellt, wobei 1 bis 4 der Reste $X_1$ bis $X_6$ jeweils eine offene Bindung darstellen und die übrigen, unabhängig voneinander, Wasserstoff oder $C_1$-$C_4$-Alkyl bedeuten;

D eine direkte Bindung; $C_1$-$C_{10}$-Alkylen; oder eine der Gruppen -CO-, -S-, -O- oder $-SO_2-$ darstellt;

$R^1$ die Bedeutung $C_1$-$C_{36}$-Alkyl; $C_3$-$C_{36}$-Alkenyl, $C_3$-$C_9$-Alkinyl; $C_1$-$C_{36}$-Alkoxy; $C_3$-$C_{36}$-Alkenyloxy; $C_5$-$C_{12}$-Cycloalkyl, welches unsubstituiert oder durch 1 bis 3 $C_1$-$C_4$-Alkyl- oder $C_1$-$C_4$-Alkoxyreste substituiert ist; $C_5$-$C_{12}$-Cycloalkoxy, welches unsubstituiert oder durch 1 bis 3 $C_1$-$C_4$-Alkyl- oder $C_1$-$C_4$-Alkoxyreste substituiert ist; Phenyl, welches unsubstituiert oder durch 1 bis 3 $C_1$-$C_4$-Alkyl- oder $C_1$-$C_4$-Alkoxyreste substituiert ist; Naphthyl, welches unsubstituiert oder durch 1 bis 3 $C_1$-$C_4$-Alkyl- oder $C_1$-$C_4$-Alkoxyreste sub-

stituiert ist; Phenoxy, welches unsubstituiert oder durch 1 bis 3 $C_1$-$C_4$-Alkyl- oder $C_1$-$C_4$-Alkoxyreste substituiert ist; Naphthoxy, welches unsubstituiert oder durch 1 bis 3 $C_1$-$C_4$-Alkyl- oder $C_1$-$C_4$-Alkoxyreste substituiert ist;

$C_7$-$C_{12}$-Phenylalkyl, welches unsubstituiert oder am Phenylring durch 1 bis 3 $C_1$-$C_4$-Alkyl- oder $C_1$-$C_4$-Alkoxyreste substituiert ist; $C_7$-$C_{12}$-Phenylalkoxy, welches unsubstituiert oder am Phenylring durch 1 bis 3 $C_1$-$C_4$-Alkyl- oder $C_1$-$C_4$-Alkoxyreste substituiert ist; oder -CO-$R^2$ hat; wobei

$R^2$ $C_1$-$C_{18}$-Alkyl; $C_5$-$C_8$-Cycloalkyl; Phenyl; Naphthyl; $C_7$-$C_9$-Phenylalkyl; oder $C_{11}$-$C_{14}$-Naphthylalkyl bedeutet; und

$R^3$ dieselben Bedeutungen umfaßt wie $R^2$ oder Wasserstoff bedeutet;

$R^4$ und $R^{4'}$, unabhängig voneinander, Wasserstoff oder $C_1$-$C_4$-Alkyl;

$R^5$, $R^6$ und $R^7$ unabhängig voneinander $C_1$-$C_3$-Alkylen;

$R^8$ Wasserstoff oder $C_1$-$C_4$-Alkyl;

$R^9$ und $R^{10}$, unabhängig voneinander, $C_3$-$C_{18}$-Alkyl oder $C_5$-$C_8$-Cycloalkyl; und $R^{11}$ $C_2$-$C_6$-Alkylen darstellen.

**3.** Verbindung der Formel I oder II gemäß Anspruch 2, worin

$A^1$ im Fall m = 1 $C_6$-$C_{18}$-Alkyl; $C_2$-$C_{12}$-Alkenyl; Cyclohexyl; Cyclohexenyl; Phenyl, das unsubstituiert oder durch 1 bis 3 Reste $C_1$-$C_4$-Alkyl oder eine Gruppe der Formel

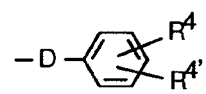

oder $C_2$-$C_4$-Alkenyl substituiert ist; $C_3$-$C_{12}$-Alkenyl, das durch -O- unterbrochen ist; oder $C_7$-$C_{12}$-Phenylalkyl, das unsubstituiert oder am Phenylring durch $C_1$-$C_4$-Alkyl oder $C_2$-$C_4$-Alkenyl oder $C_1$-$C_4$-Alkoxy substituiert ist, darstellt;

$A^1$ im Fall m = 2 eine direkte Bindung; $C_1$-$C_{18}$-Alkylen; $C_2$-$C_{18}$-Alkylen, das durch -O- oder -S- unterbrochen ist; $C_2$-$C_{18}$-Alkenylen; $C_5$-$C_9$-Cycloalkylen; Phenylen, das unsubstituiert oder durch 1 oder 2 Reste $C_1$-$C_4$-Alkyl oder $C_1$-$C_4$-Alkoxy und/oder eine Gruppe der Formel

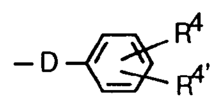

oder $C_2$-$C_4$-Alkenyl substituiert ist; einen zweiwertigen Rest der Formel

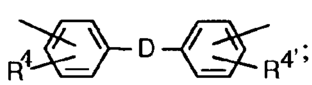

$A^1$ im Fall m = 3 $C_3$-$C_8$-Alkantriyl; durch eine tertiäre Hydroxylgruppe substituiertes $C_3$-$C_8$-Alkantriyl; Benzoltriyl; $N[(CH_2)-]_3$; $C_5$-$C_8$-Cycloalkantriyl; oder 1,3,5-Triazin-2,4,6-triyl;

$A^1$ im Fall m = 4 einen Benzol-, Cyclopentyl- oder Cyclohexylrest mit 4 freien Valenzen; oder

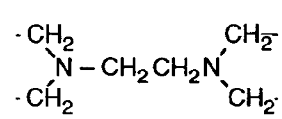

bedeutet;

$A^2$ im Fall n = 1 Phenyl, das unsubstituiert oder durch $C_1$-$C_4$-Alkyl oder eine Gruppe der Formel

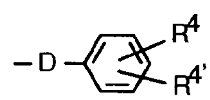

oder $C_2$-$C_4$-Alkenyl substituiert ist; oder Naphthyl bedeutet;

$A^2$ im Fall n = 2 Phenylen, das unsubstituiert oder durch $C_1$-$C_4$-Alkyl oder eine Gruppe der Formel

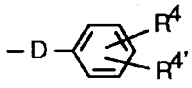

substituiert ist; einen zweiwertigen Rest der Formel

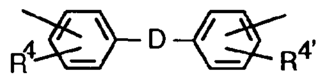

oder Naphthylen;
$A^2$ im Fall n = 3 Benzoltriyl;
$A^2$ im Fall n = 4 einen Benzolrest mit 4 freien Valenzen bedeutet;
oder $A^2$ einen n-wertigen Triphenyltriazinylrest der Formel

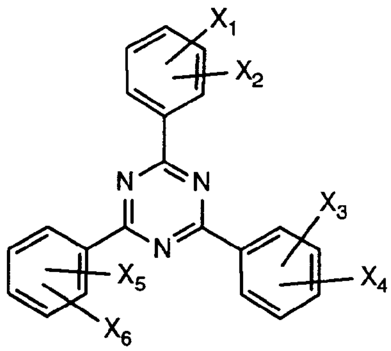

darstellt, wobei 1 bis 4 der Reste $X_1$ bis $X_6$ jeweils eine offene Bindung darstellen und die übrigen, unabhängig voneinander, Wasserstoff oder Methyl bedeuten;
D eine direkte Bindung oder eine der Gruppen -CO-, -S-, -O-, -$SO_2$- oder

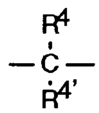

darstellt;
$R^1$ die Bedeutung $C_1$-$C_{18}$-Alkyl; $C_4$-$C_{22}$-Alkoxy; $C_3$-$C_8$-Alkenyl; $C_3$-$C_8$-Alkinyl; $C_5$-$C_8$-Cycloalkyl; $C_5$-$C_8$-Cycloalkoxy; $C_7$-$C_{12}$-Phenylalkyl; $C_7$-$C_{12}$-Phenylalkoxy; $C_1$-$C_8$-Alkanoyl; $C_3$-$C_5$-Alkenoyl; oder Benzoyl hat; und
$R^4$ und $R^{4'}$, unabhängig voneinander, Wasserstoff oder $C_1$-$C_4$-Alkyl darstellen.

**4.** Verbindung der Formel I oder II gemäß Anspruch 3, worin
m und n jeweils ganze Zahlen aus dem Bereich 1 bis 4 sind;
$A^1$ im Fall m = 1 $C_6$-$C_{18}$-Alkyl;
$A^1$ im Fall m = 2 $C_2$-$C_{18}$-Alkylen; Cyclohexylen; Phenylen; einen zweiwertigen Rest der Formel

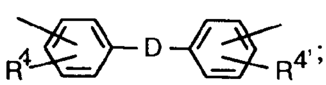

$A^1$ im Fall m = 3 Benzoltriyl oder Cyclohexantriyl; und
$A^1$ im Fall m = 4 einen Benzolrest mit 4 freien Valenzen bedeutet;
$A^2$ im Fall n = 1 Phenyl, das unsubstituiert oder durch $C_1$-$C_4$-Alkyl substituiert ist; oder Naphthyl bedeutet;
$A^2$ im Fall n = 2 Phenylen, das unsubstituiert oder durch $C_1$-$C_4$-Alkyl oder eine Gruppe der Formel

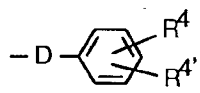

substituiert ist; Naphthylen; oder einen zweiwertigen Rest der Formel

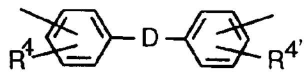

bedeutet;
$A^2$ im Fall n = 3 Benzoltriyl; und
$A^2$ im Fall n = 4 einen Benzolrest mit 4 freien Valenzen darstellt;
D eine der Gruppen $-CH_2-$, -CO- oder

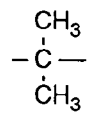

darstellt; und
$R^4$ und $R^{4'}$, unabhängig voneinander, Wasserstoff, Methyl oder tert.-Butyl darstellen.

**5.** Verbindung der Formel I oder II gemäß Anspruch 4, worin m eine ganze Zahl aus dem Bereich 1 bis 4 und n eine ganze Zahl aus dem Bereich 1 bis 3 ist;
$A^1$ im Fall m = 1 $C_{10}$-$C_{18}$-Alkyl;
$A^1$ im Fall m = 2 $C_2$-$C_{18}$-Alkylen; Cyclohexylen; oder Phenylen;
$A^1$ im Fall m = 3 Benzoltriyl oder Cyclohexantriyl bedeutet; und
$A^1$ im Fall m = 4 Benzoltetrayl ist;
$A^2$ im Fall n = 1 Phenyl;
$A^2$ im Fall n = 2 Phenylen oder einen zweiwertigen Rest der Formel

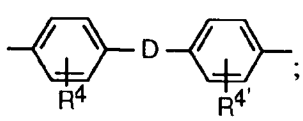

;

und
$A^2$ im Fall n = 3 Benzoltriyl bedeutet;
D eine der Gruppen -CO- oder

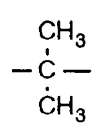

darstellt;
$R^1$ die Bedeutung $C_1$-$C_6$-Alkyl; $C_6$-$C_{12}$-Alkoxy; Cyclohexyl; Cyclohexyloxy; Cycloheptyloxy; Cyclooctyloxy; oder $C_7$-$C_9$-Phenylalkyl hat; und
$R^4$ und $R^{4'}$, unabhängig voneinander, Wasserstoff, Methyl oder tert.-Butyl darstellen.

**6.** Verbindung der Formel I oder II gemäß Anspruch 3, worin m 1 oder 2 und n 1 ist;
$A^1$ im Fall m = 1 $C_2$-$C_{12}$-Alkenyl; Cyclohexylen; Phenyl, das durch $C_2$-$C_4$-Alkenyl und optional durch $C_1$-$C_4$-Alkyl oder eine Gruppe der Formel

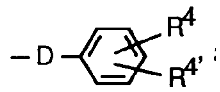

substituiert ist;
$C_3$-$C_{12}$-Alkenyl, das durch -O- unterbrochen ist; oder $C_7$-$C_{12}$-Phenylalkyl, das am Phenylring durch $C_2$-$C_4$-Alkenyl substituiert ist;
$A^1$ im Fall m = 2 $C_2$-$C_{18}$-Alkenylen; oder Phenylen, das am Phenylring durch $C_2$-$C_4$-Alkenyl substituiert ist;
$A^2$ im Fall n = 1 Phenyl, das durch $C_2$-$C_4$-Alkenyl substituiert ist;
D eine direkte Bindung oder eine der Gruppen -O- oder

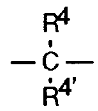

darstellt;
$R^1$ die Bedeutung $C_1$-$C_{18}$-Alkyl; $C_4$-$C_{22}$-Alkoxy; $C_5$-$C_8$-Cycloalkyl; $C_5$-$C_8$-Cycloalkoxy; $C_7$-$C_{12}$-Phenylalkyl; $C_1$-$C_8$-Alkanoyl; oder Benzoyl hat; und
$R^4$ und $R^{4'}$, unabhängig voneinander, Wasserstoff oder $C_1$-$C_4$-Alkyl darstellen.

**7.** Zusammensetzung enthaltend (a) ein gegen oxidativen, thermischen oder/und aktinischen Abbau empfindliches organisches Material und (b) mindestens eine Verbindung der Formeln I und/oder II gemäß Anspruch 1.

**8.** Zusammensetzung gemäß Anspruch 7, worin das organische Material ein synthetisches organisches Polymer oder ein Gemisch synthetischer organischer Polymere ist.

**9.** Zusammensetzung gemäß Anspruch 8, worin Komponente (a) eine Überzugszusammensetzung ist.

**10.** Zusammensetzung gemäß Anspruch 7, worin Komponente (b) in einer Menge von 0,01 bis 10 % bezogen auf das Gesamtgewicht der stabilisierten Zusammensetzung enthalten ist.

**11.** Zusammensetzung gemäß Anspruch 7, worin neben den Komponenten (a) und (b) zusätzlich (c) mindestens ein herkömmliches Additiv enthalten ist.

**12.** Verwendung von Verbindungen der Formeln I und/oder II zum Stabilisieren von organischem Material gegen oxidativen, thermischen oder aktinischen Abbau.

**13.** Verfahren zum Stabilisieren von organischem Material gegen thermischen, oxidativen oder/und aktinischen Abbau, dadurch gekennzeichnet, daß man diesem Material mindestens eine Verbindung der Formeln I und/oder II zusetzt.

**14.** Verfahren zur Herstellung einer Verbindung der Formel I oder der Formel II gemäß Anspruch 1, dadurch gekennzeichnet, daß man eine Piperidinverbindung der Formel IV

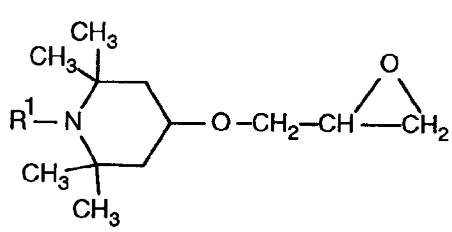

(IV)

(a) durch Reaktion mit einer Carbonsäure der Formel V

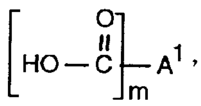

(V)

zum Ester der Formel I, oder
(b) durch Reaktion mit einem Phenol der Formel VI

$$\left[HO\right]_n - A^2 \qquad \text{(VI)}$$

zum Phenolether der Formel II umsetzt.

Europäisches Patentamt

# EUROPÄISCHER RECHERCHENBERICHT

Nummer der Anmeldung
EP 94 81 0397

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 058 434 (ADEKA ARGUS CHEMICAL CO., LTD.)<br>* Ansprüche 1,5-7 * | 1,7,8,12 | C07D211/46<br>C07D405/12<br>C08K5/3435 |
| A | PATENT ABSTRACTS OF JAPAN<br>vol. 8, no. 283 (C-258) 25. Dezember 1984<br>& JP-A-59 152 934 (ADEKA ARGUS KAGAKU KK) 31. August 1984 | 1,7,8,12 | |
| A | EP-A-0 153 907 (CIBA-GEIGY)<br>* Zusammenfassung; Ansprüche 1,5-9 * | 1,7,8, 11,12 | |
| A | EP-A-0 518 807 (CIBA-GEIGY)<br>* Zusammenfassung; Ansprüche 1,8-13 * | 1,7,8,12 | |
| A,D | EP-A-0 097 616 (CIBA-GEIGY)<br>* Seite 17, letzte Verbindung *<br>* Zusammenfassung; Anspruch 1 * | 1,12 | |
| A,D | DIE MAKROMOLEKULARE CHEMIE - MACROMOLECULAR SYMPOSIA,<br>Bd.27, 1989<br>Seiten 231 - 237<br>J. LUSTON ET AL. 'Functional derivatives of sterically hindered amines. 1. Glycidyl compounds'<br>* Seite 233, Schema 2 und Tabelle 1 * | 1,12 | RECHERCHIERTE SACHGEBIETE (Int.Cl.6)<br>C07D<br>C08K |

Der vorliegende Recherchenbericht wurde für alle Patentansprüche erstellt

| Recherchenort | Abschlußdatum der Recherche | Prüfer |
|---|---|---|
| BERLIN | 12. Oktober 1994 | Hass, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)